# EUROPEAN PATENT SPECIFICATION

(11) **EP 0 788 486 B1**
(45) Date of publication and mention of the grant of the patent: **12.07.2000**
(21) Application number: 94931213.6
(22) Date of filing: 24.10.1994
(51) Int. Cl.: C07D 239/42, C07D 401/14, C07D 213/73, C07C 311/29, A01N 47/36

(54) **HERBICIDAL SULFONYL UREA DERIVATIVES**
HERBIZIDE SULFONYLHARNSTOFF-DERIVATE
DERIVES HERBICIDES DE SULFONYLUREE

(43) Date of publication of application: 13.08.1997
(73) Proprietor: KOREA RESEARCH INSTITUTE OF CHEMICAL TECHNOLOGY, Daejeon, 305-343 (KR)
(72) Inventor: KIM, Dae Whang, Kongdong Apartment 6-303, Daejeon 305-340 (KR); KO, Young Kwan, Hanbit Apartment 102-1702, Daejeon 305-333 (KR); CHANG, Hae Sung, Hanbit Apartment 105-201, Daejeon 305-333 (KR); RYU, Jae Wook, Hanbit Apartment 120-305, Daejeon 305-333 (KR); JO, In Ho, 155, Sinsung-dong, Daejeon 305-345 (KR); WOO, Jae Chun, Kyungnam Apartment 1-406, Daejeon 302-162 (KR); KU, Dong Whan, Hanbit Apartment 128-604, Daejeon 305-333 (KR); KIM, Jin Seog, Shindonga Apartment 10-601, Daejeon 300-200 (KR)
(74) Representative: Weber, Dieter, Dr.
(86) International application number: KR9400147
(87) International publication number: WO9612708

(56) References cited:
- EP-A- 0 044 807
- EP-A- 0 240 216
- EP-A- 0 512 953
- WO-A-92/14728
- WO-A-92/15568
- US-A- 4 443 245
- US-A- 4 532 328

## Description

### BACKGROUND OF THE INVENTION

### Field of the Invention

The present invention relates to novel sulfonyl urea derivatives of the following formula (I) having erythro-type stereoisomer as herbicides for treatment of pre-emergence and/or post-emergence, their use and composition as agriculturally suitable herbicides. 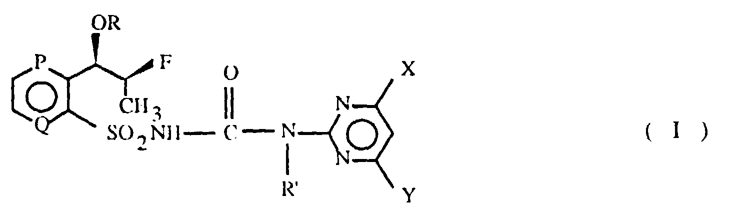 wherein,
P and Q, as equivalent or different group respectively, are CH or N, and present as aromatic ring including P and Q as benzene or pyridine ring ;
R is H, 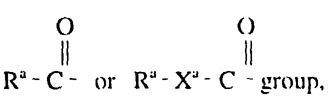 wherein R^{a} is C₁∼C₄ alkyl, C₁∼C₃ haloalkyl, C₂∼C₄ alkenyl or C₂∼C₄ alkynyl group, wherein X^{a} is O, S, NH or NR^{a} group;
R' is H or CH₃ group; and
X and Y are independently halogen atom, C₁∼C₂ alkyl, C₁∼C₂ alkoxy or C₁∼C₂ haloalkoxy group.

### Description of the Prior Art

It is publicly well-known that sulfonyl urea derivatives possess a herbicidal activity. Such examples containing sulfonyl urea are;
(1) Korea Patent publication No. 93-9825 WO-A-9215568) discloses the compound having the following formula(A) 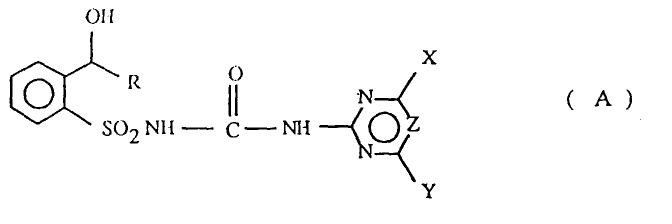 wherein,
   R is haloalkyl;
   X and Y are independently CH₃, OCH₃ or Cl etc.;
   Z is CH or N.
(2) Korea Patent publication No. 93-9507 (WO-A-9214728) discloses the compound having the following formula(B) 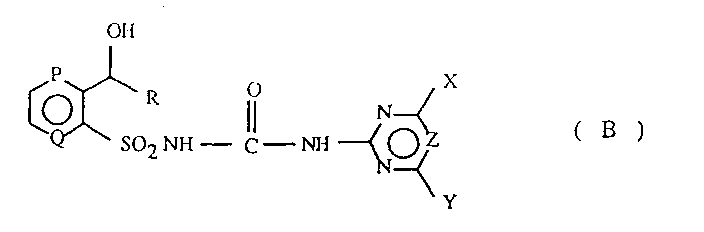 wherein,
   R, X, Y and Z are as previously defined,
   P and Q are differently N or CH.

If R group of the above formula(A) and (B) includes asymmetric carbon atom, then the above compound has two stereoisomers which are threo- and erythro-type by reason of two asymmetric carbon atom. But herbicidal activity and selectivity of the above stereoisomers have been not disclosed.

### SUMMARY OF THE INVENTION

The object of the present invention is to provide novel sulfonyl urea derivatives having very prominent good selectivity toward rice and wheat and also possess a herbicidal activities for annual and perennial weed, especially a barnyard grass.

Another object of this invention is to provide herbicidal compositons containing said derivatives as active compounds.

### BRIEF DESCRIPTION OF THE INVENTION

- Fig. 1: is stereoconfiguration based upon X-ray crystallography analysis of the compound manufactured by EXAMPLE 1.
- Fig. 2: is stereoconfiguration based upon X-ray crystallography analysis of the compound manufactured by EXAMPLE 9.

### DETAILED DESCRIPTION OF THE INVENTION

The present invention relates to herbicidal sulfonyl urea derivatives with substituent of erythro-type stereoisomer having the following formula(I), which have herbicidal selectivity toward rice and wheat, and their agriculturally suitable salts. 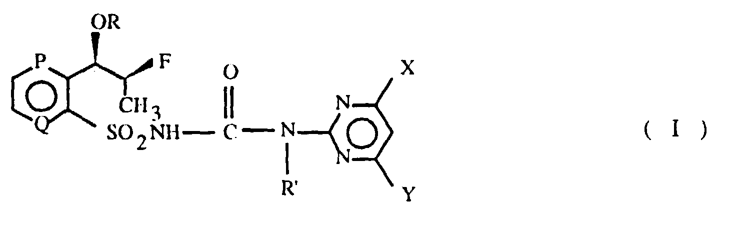 wherein,
P, Q, R, R', X and Y are as previously defined.

A preferred group of erythro-type stereoisomer of the above formula(I), in view of a strong activity and a good selectivity is as follows :
(1) Benzene(P and Q are independently CH)
(2) Pyridine(P is N, and Q is CH)
(3) R is hydrogen atom
(4) R' is hydrogen atom
(5) R is acetyl group
(6) X and Y are methoxy group.

These compounds can easily control barnyard grass as well as a perennial weed causing trouble for rice and can be used agriculturally as herbicidal composition for rice. Especially the following compounds have a good selectivity for rice :
Erythro *N*-[(4,6-dimethoxy-pyrimidin-2-yl)aminocarbonyl]-2-(2-fluoro-1-hydro-xy-n-propyl)-3-pyridinesulfonamide,
Erythro *N*-[(4,6-dimethoxy-pyrimidin-2-yl)aminocarbonyl]-2-(2-fluoro-1-hydro-xy-*n*-propyl)-benzenesulfonamide, etc..

The erythro-type compounds of the above formula(I) according to the present invention have more prominent herbicidal activity than threo-type or mixture of erythro-and threo-type. Furthermore, the erythro-type compounds of the above formula(I) may be used as herbicides or active ingredient of herbicidal composition because of a good selectivity for rice and wheat.

A pure compound of erythro-type having the above formula(I) according to the present invention can be prepared by reactions described in herein below, but should not be constructed to be limited hereto.

The compound of the above formula(I), in which R is hydrogen atom, can be obtained by hydrolyzing the compound of the above formula(I), where R is acyl group such as acetyl group, in present of alkali.

In order to hydrolyze the above acyl group, alkali such as LiOH, KOH, NaOH, Li₂CO₃, Na₂ CO₃, K₂CO₃, etc., preferably LiOH, may be used.

The above hydrolysis reaction is carried out under water or organic solvent, as a mixture of water with unreacting solvent such as methanol, ethanol, acetone, tetrahydrofuran, dimethylformamide, etc., or solvent alone. The hydrolysis occurs at the temperature of 0∼80°C in a reaction time of 1∼24 hours, and then the obtained product may be easily seperated by acidifying with aqueous HCl solution.

As an other process, after acidifying, the obtained product is extracted with methylene chloride, ethyl acetate, etc. and then concentrated to obtain the final product. If necessary, a pure product can be obtained by purification using HPLC.

The hydrolysis in the above reaction is carried out as shown in the following reaction scheme. 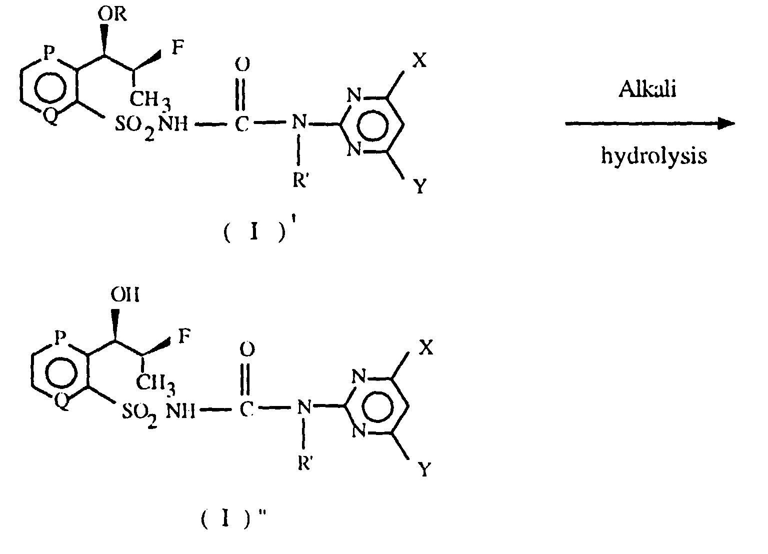 wherein,
P, Q, R', X and Y are respectively defined as the above formula (I), and
R is defined as the above formula (I) except of hydrogen atom.

Also, the compounds of the above formula (I) according to the present invention can be prepared by reacting the erythro-type compound having the following formula (II) with the compound having the follwoing formula (III). 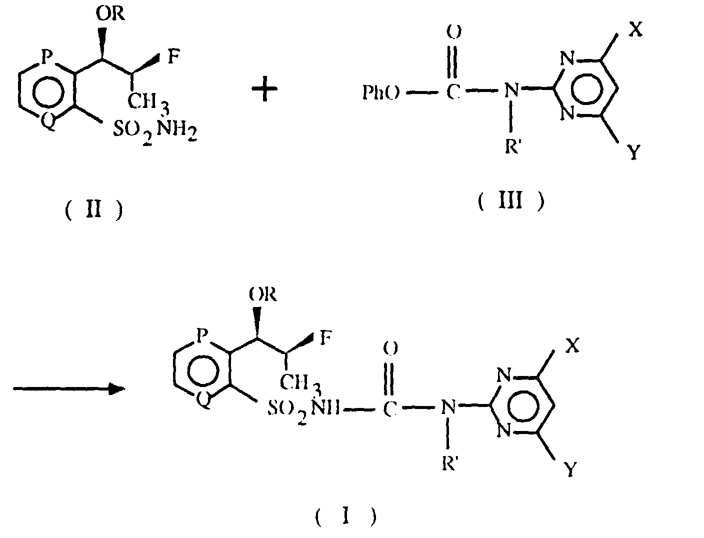 wherein,
P, Q, R, R', X and Y are respectively defined as the above formula(I).

In the above reaction, unreacting solvent such as tetrahydrofuran, acetone, acetonitrile, dioxane, methylene chloride, toluene, butanone, pyridine, dimethylformamide, etc., may be used.

The reaction may be preferably carried out under strong base such as DBU or DABCO, etc. in a small quantity at the temperature of 20∼80°C. The above reaction is referred to in U.S. patent No. 4,443,245 and thereafter the desired product can be obtained by acidifying by the method mentioned in European Patent No. 44,807. If necessery, a pure product can be obtained by purification by HPLC. Said, DBU represents 1,8 - diazabicyclo[5.4.0] undec-7-ene, and DABCO represents 1,4-diazabicyclo [2.2.2]octane.

Also, the compound of the formula(III) used for preparing the above formula(I) can be easily obtained by the prior art.

On the other hand, the erythro-type of the above formula(II) can be prepared by the following reaction scheme. 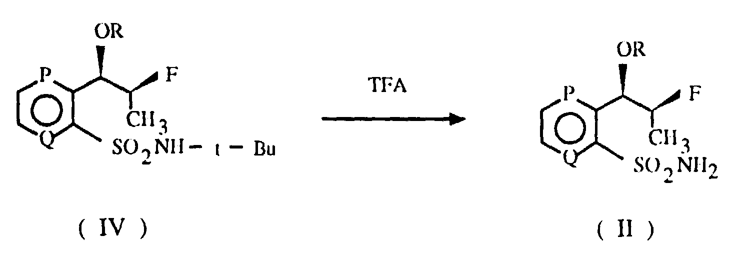 wherein,
P, Q and R are respectively defined as the above.

In the above reaction, the primary sulfonamide of erythro-type having the above formula(II) can be prepared by treating *N-t*-butylsulfonamide of the above formula(IV) with an acid such as trifluoroacetic acid (TFA) at the temperature of 0∼50°C.

Also, the erythro-type of the above formula(IV) used in the above reaction can be prepared by common acylation of the following formula(V). The pure erythro-type of the above formula(IV) can be seperated from mixture of threo- and erythro-type by purification such as column chromatograph, HPLC or prep-TLC.

The compound of the following formula(V) can be prepared by selective reduction of the compound of the following formula(VI) with selective reductant such as diisobutylaluminum hydride. 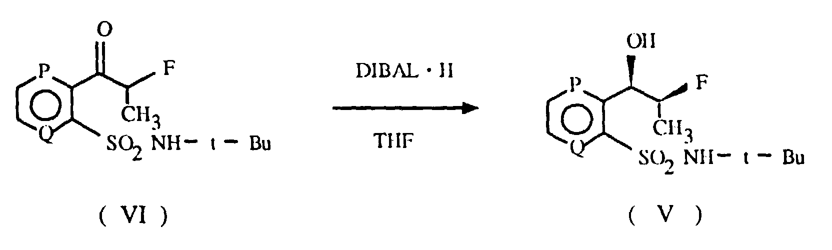 wherein,
P and Q are respectively defined as the above.
DIBAL · H is diisobutylaluminum hydride.

In the above reaction, preferably P is N and Q is CH.

The pure erythro-type of the above formula(V) can be easily purified using column chromatograph.

The compound of the above formula(IV) can also be prepared by another process as shown in the following reaction. 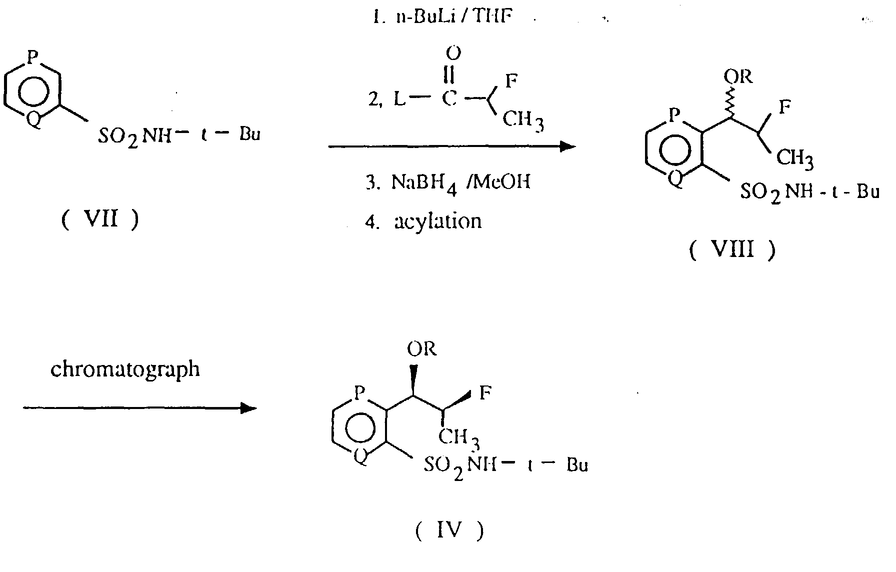 wherein,
P and Q are respectively defined as the above formula(I),
R is defined as the above formula(I) except of hydrogen atom,
L is alkoxy, N(CH₃)₂ or NCH₃(OCH₃), etc..

The above reaction process has been disclosed in Korea Patent Application No. 91-3704 and No. 91-3014. *n*-Butyl lithium of 2 equivalents are added in the compound of the above formula(VII) in THF solvent for 1∼24 hours at -80∼+30°C to obtain dilithio salt, and then 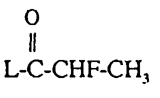 is added at -70∼-80°C to obtain ketone compound. Hydroxy compound is obtained by reduction of the ketone compound with NaBH₄ , and then the compound of formula VIII wherein R is acetyl group is obtained by acylation under acetic anhydride, DMAP and pyridine.

The pure erythro-type of the above formula (IV) can be easily obtained by separat ion and purification techniques such as HPLC, column chromatograph, prep-TLC, etc..

On the other hand, salts of the compound of the above formula(I) which are also useful as herbicide, can be prepared by various methods according to prior art. For example, metal salts of the compound can be prepared by reacting the above formula(I) compound with strong basic anion, e.g. alkali or alkaline earth metal solution having hydroxyl group, alkoxide or carbonate, and also quaternary amine salt alike.

A salt of the formula(I) compound may also be obtained by cation exchange. The cation exchange can be carried out by directly reacting a solution containing cation for exchange with the solution of salt of formula(I), for example aqueous solution of alkali metal or quaternary amine salt. This method is useful when the desirable salt is water soluble, especially sodium, potassium or calcium salt.

The above manufacturing methods are summarized briefly, and the methods can be carried out easily by a person skilled in the technical field for manufacturing sulfonyl urea or organic composition.

The compounds of the above formula(I) according to the present invention may be specified as the following Table 1.

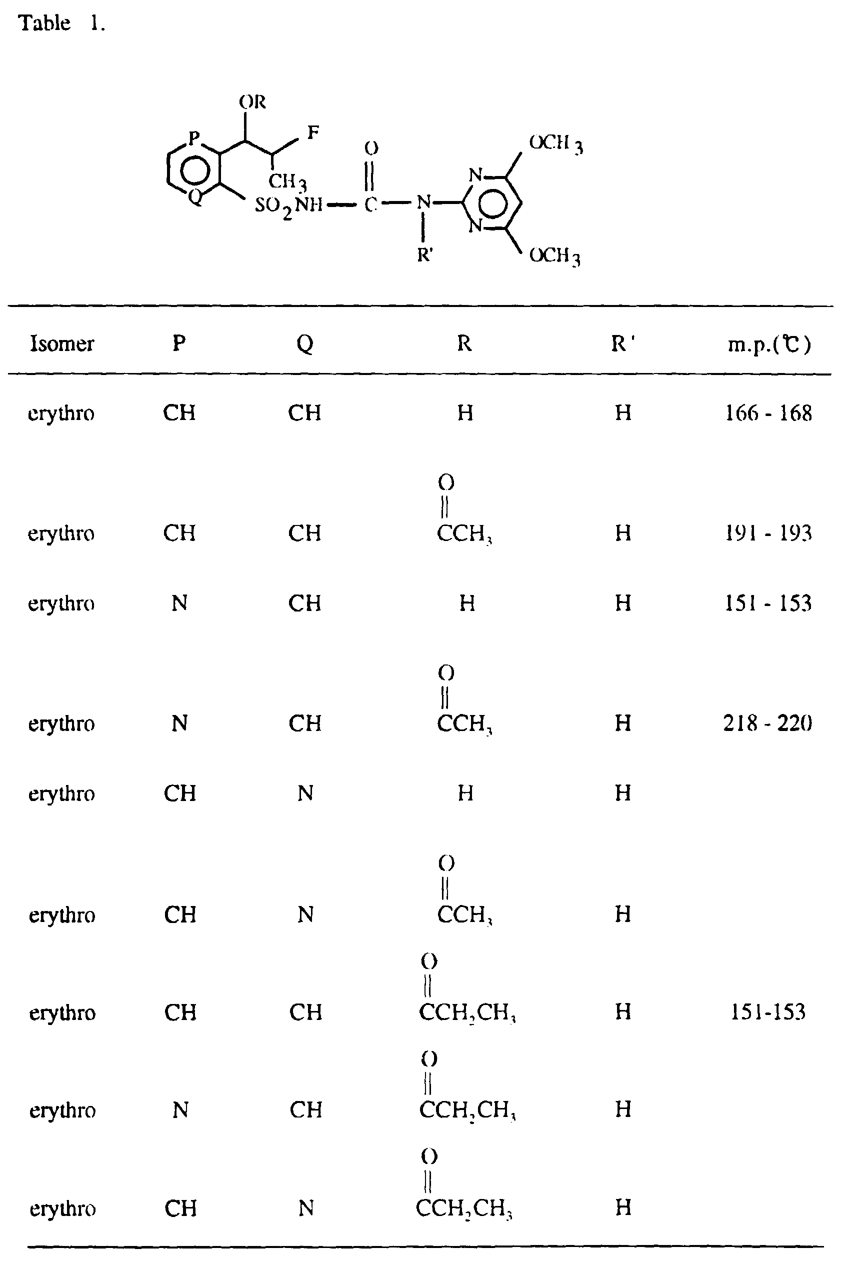

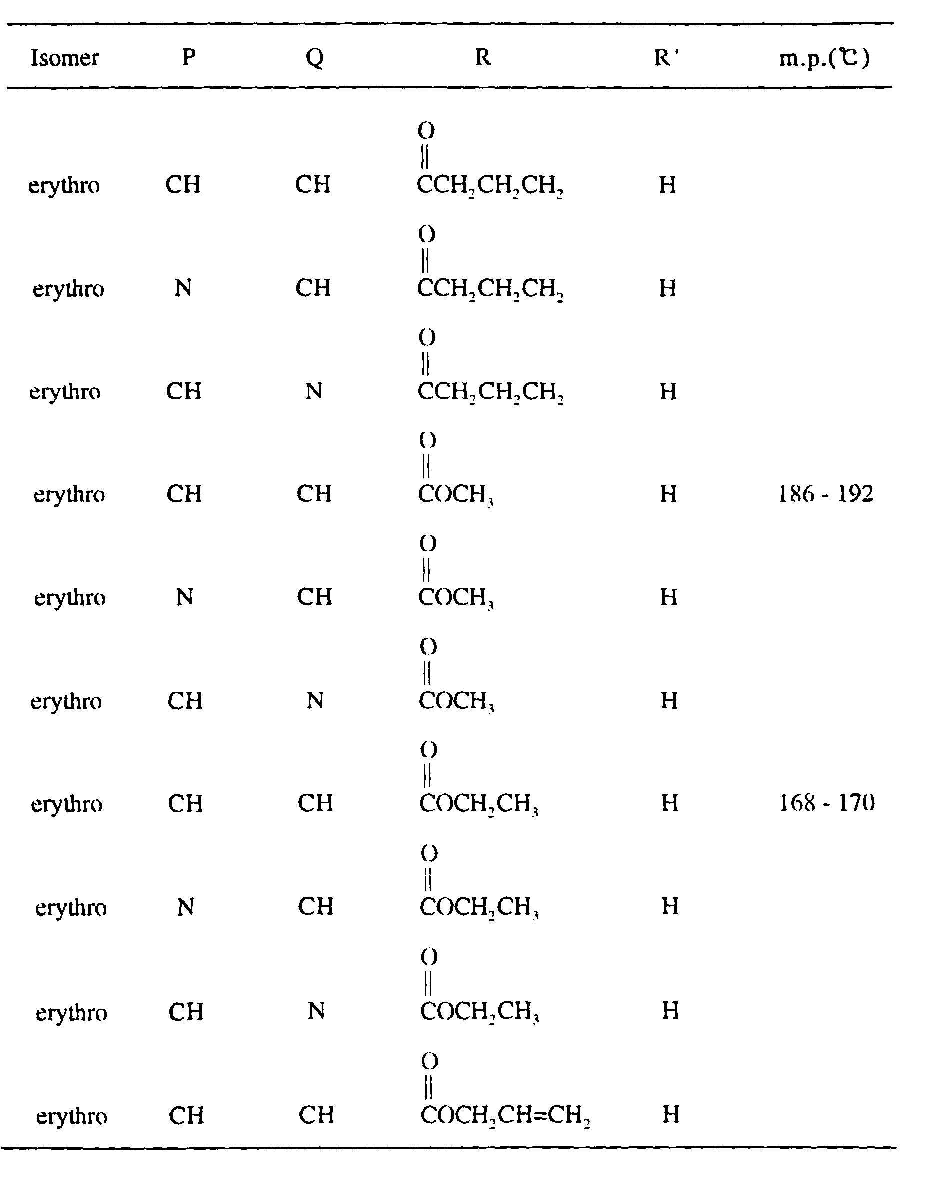

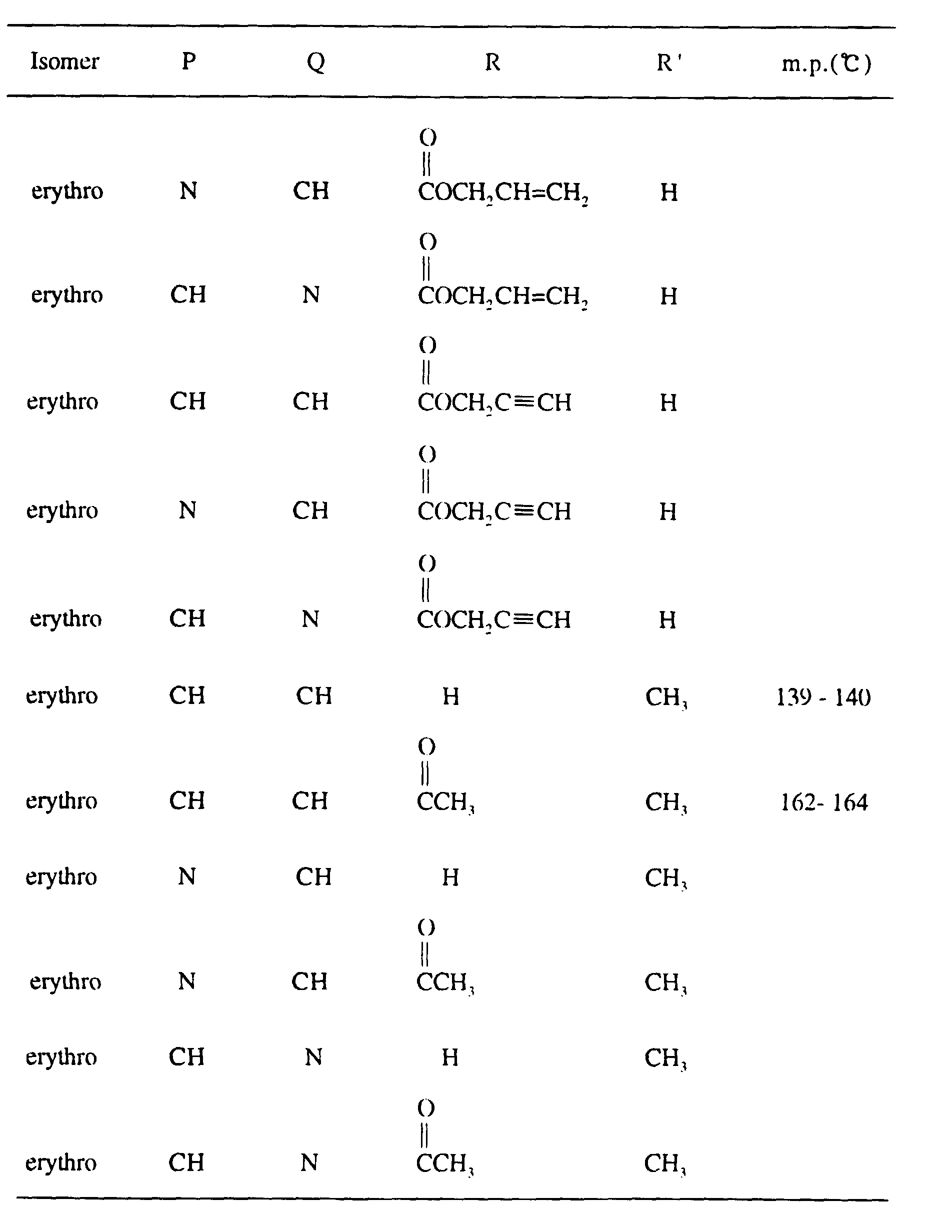

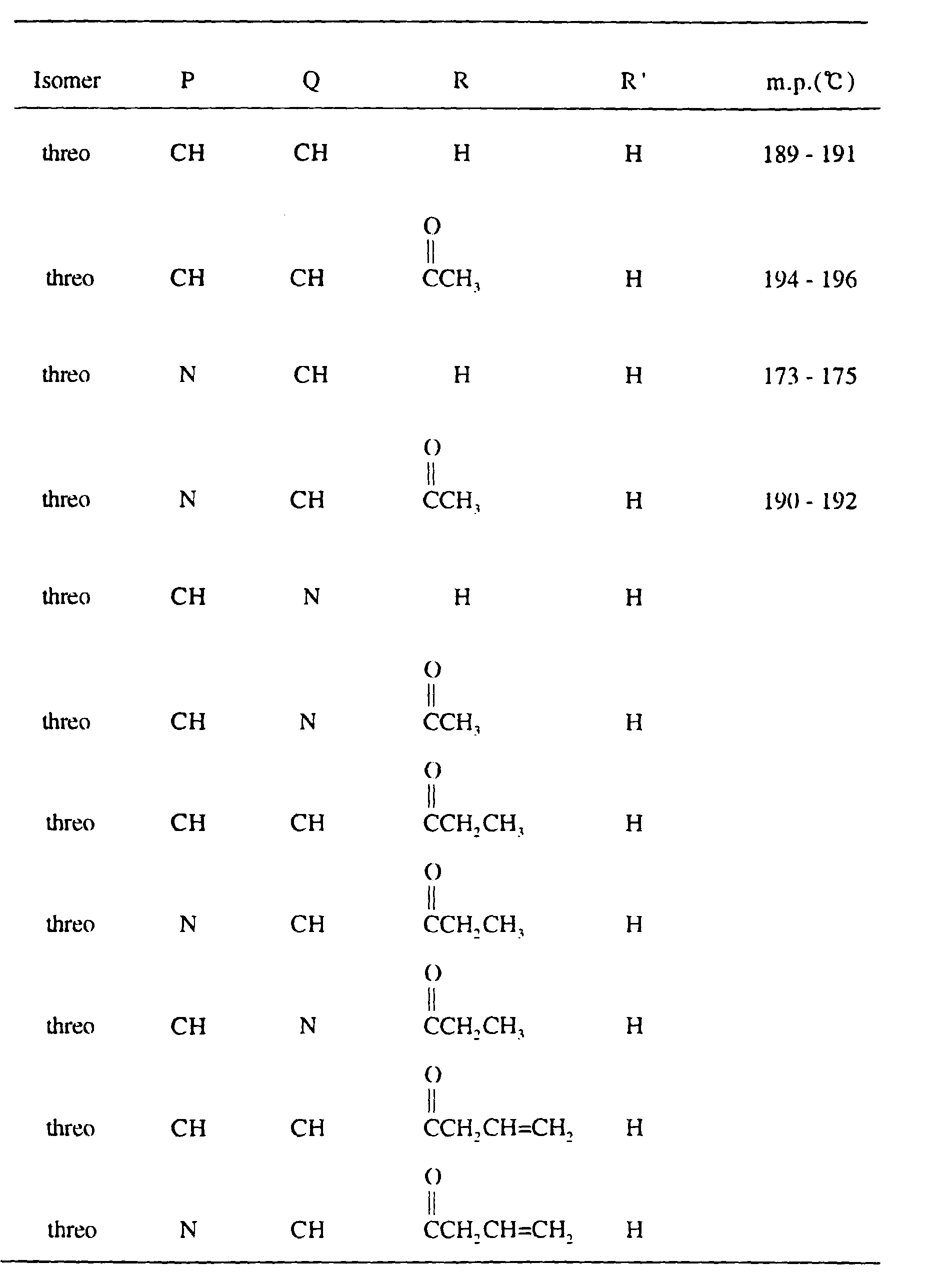

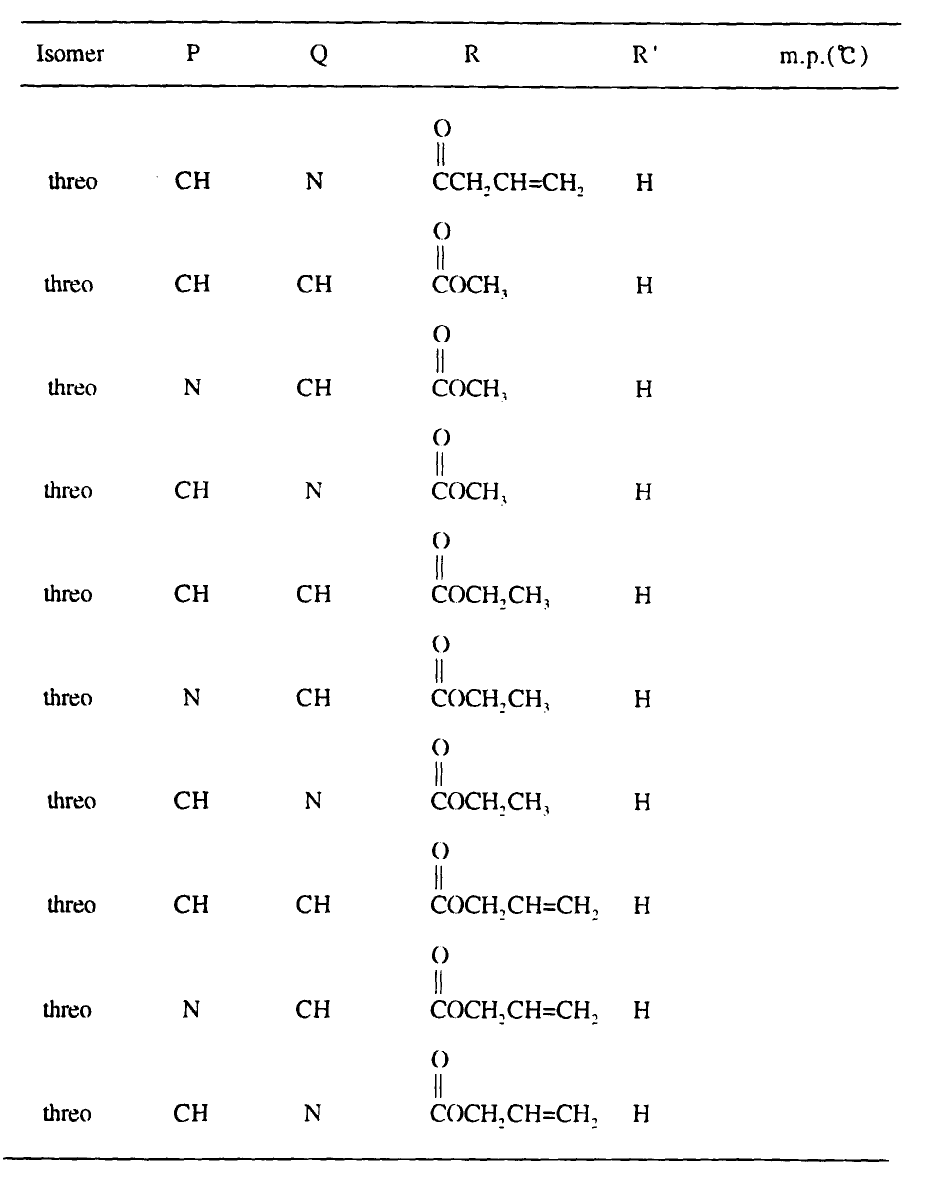

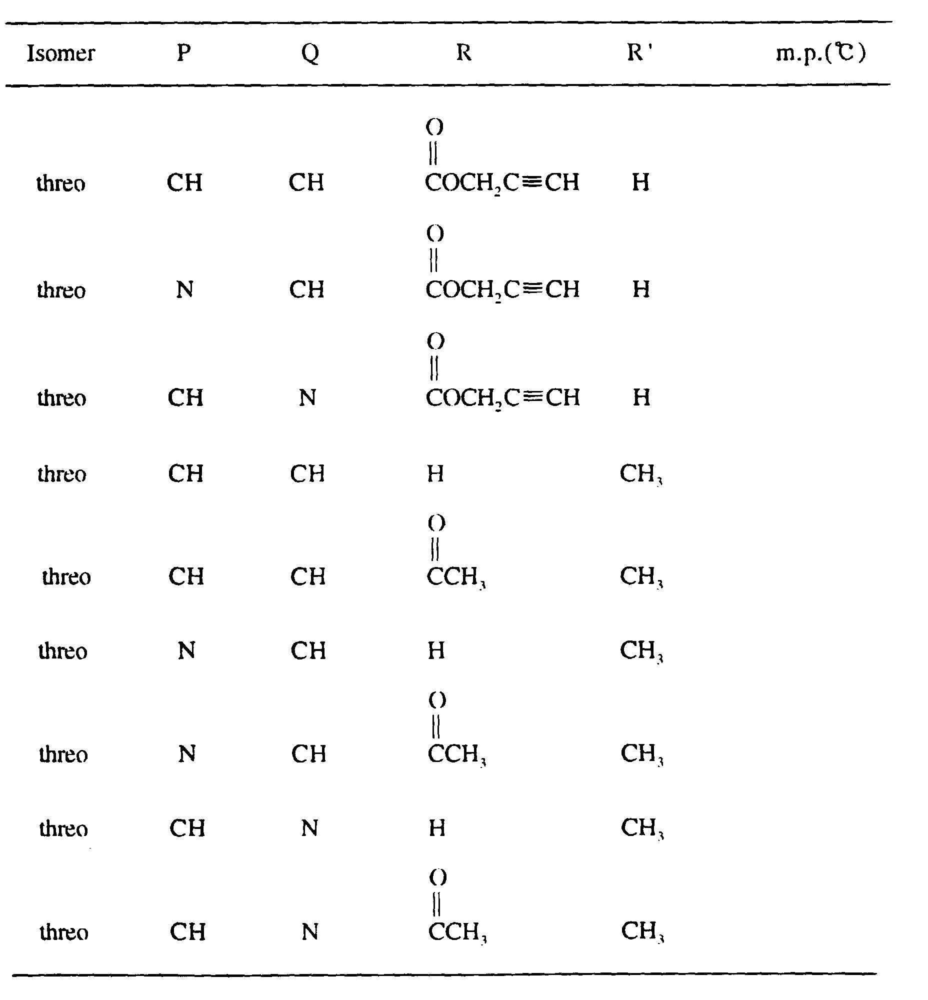

The sulfonyl urea derivatives having erythro-type stereoisomer of the above formula(I) according to the present invention are useful as herbicides. The applied method is given below.

### [Utility]

The compounds according to the present invention represent very high activity as pre- or post- emergence herbicides and water surface treatment or leaf treatment herbicides for rice.

The used amount of compound of the present invention is decided by several factor, that is, kinds of weeds, climate or weather, formulations selected, the applied method or the size of weed etc.

The active ingredients can be generally used from 1 g to 1 kg per hectare. Smaller quantity may be used in soil containing low organic matter or sandy soil, young plant or when the herbicidal effect is need of short-termed duration.

The compounds according to the present invention are especially effective as ingredient for control of weed in rice and wheat field, especially leaf-width weed, graminaceae weed and annual or perennial weed. The compounds are particularly effective for control of barnyard grass.

The list of weeds controllable by the compounds of the present invention is given below.

### [the list of weeds]

### dicotyledon weeds genus:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Arbutilon, Emex, Datura, Viola, Galeopsis, Papaver, Centaurea.

### monocotyledon weeds genus:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Dactyloctenium, Agrostis, Alopecurus, Apera, Heteranthera, Leptochloa.

The compounds of the present invention can be used as alone or in combination with two, three or four additives with other herbicides. The appropriate herbicides for mixed-using with the compounds of the present invention are given bleow. It is particularly useful for control of weeds to use the mixture of the compounds of the present invention and the below herbicides.

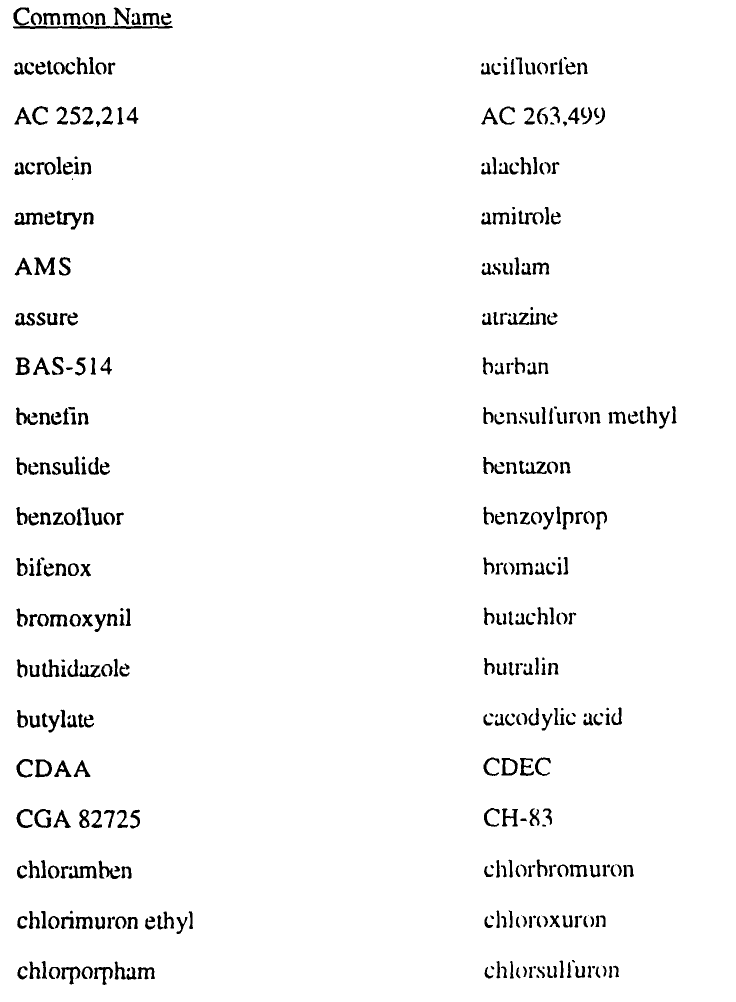

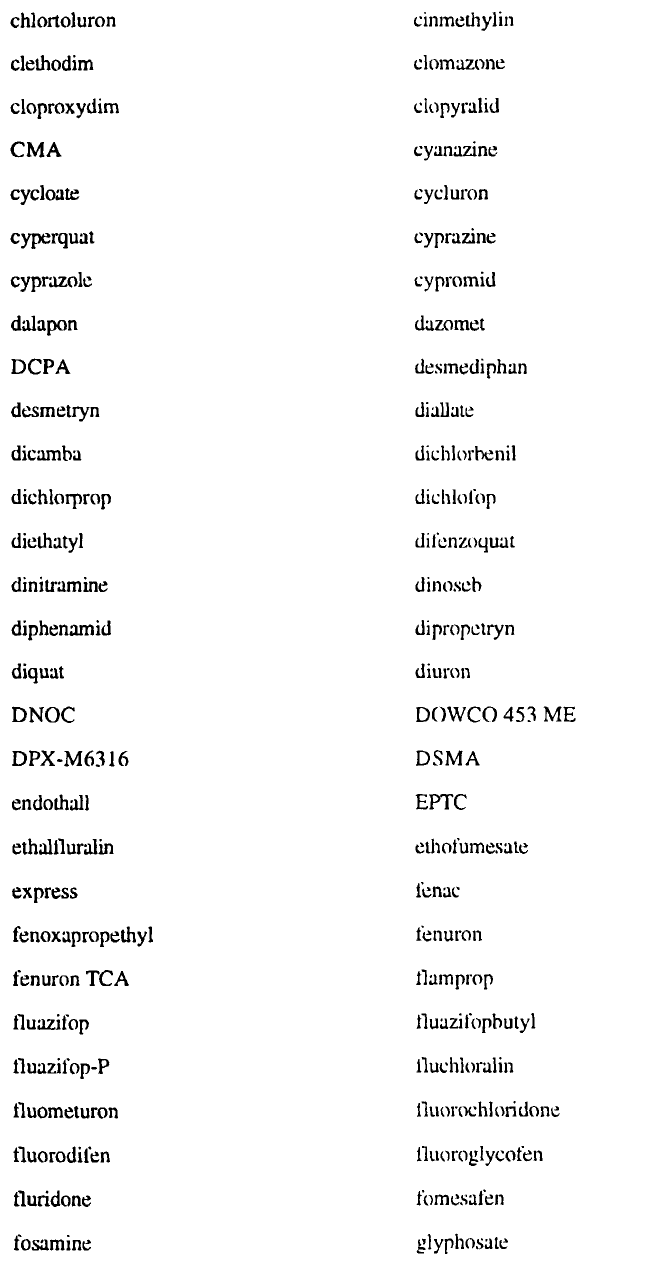

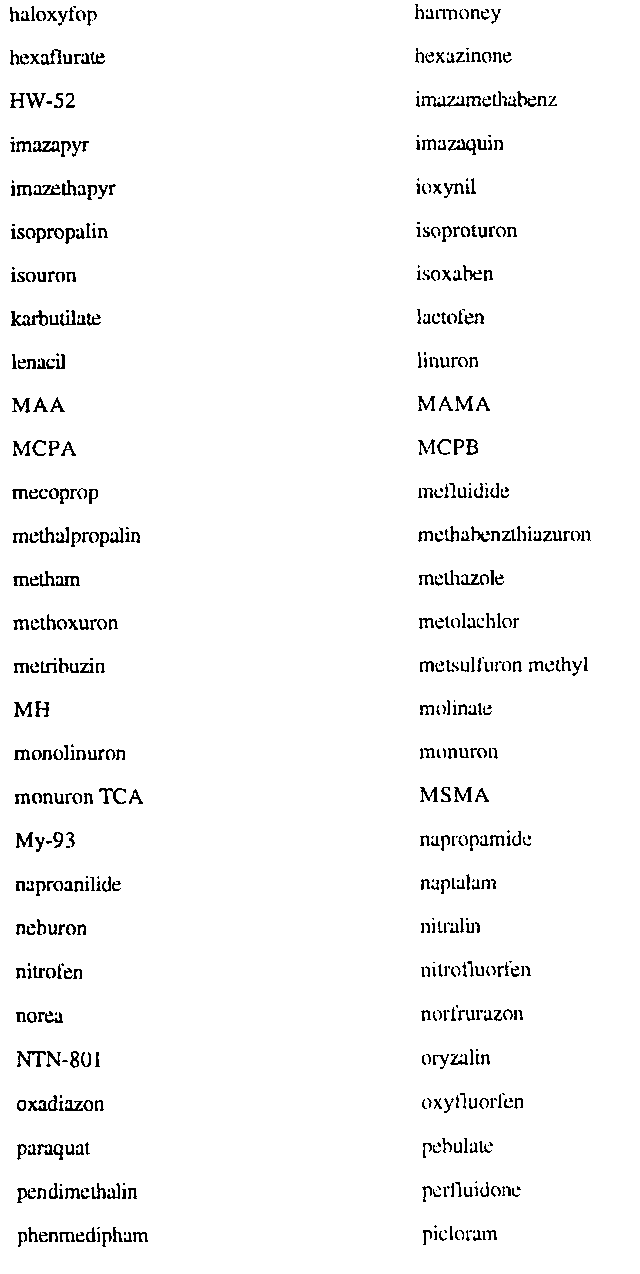

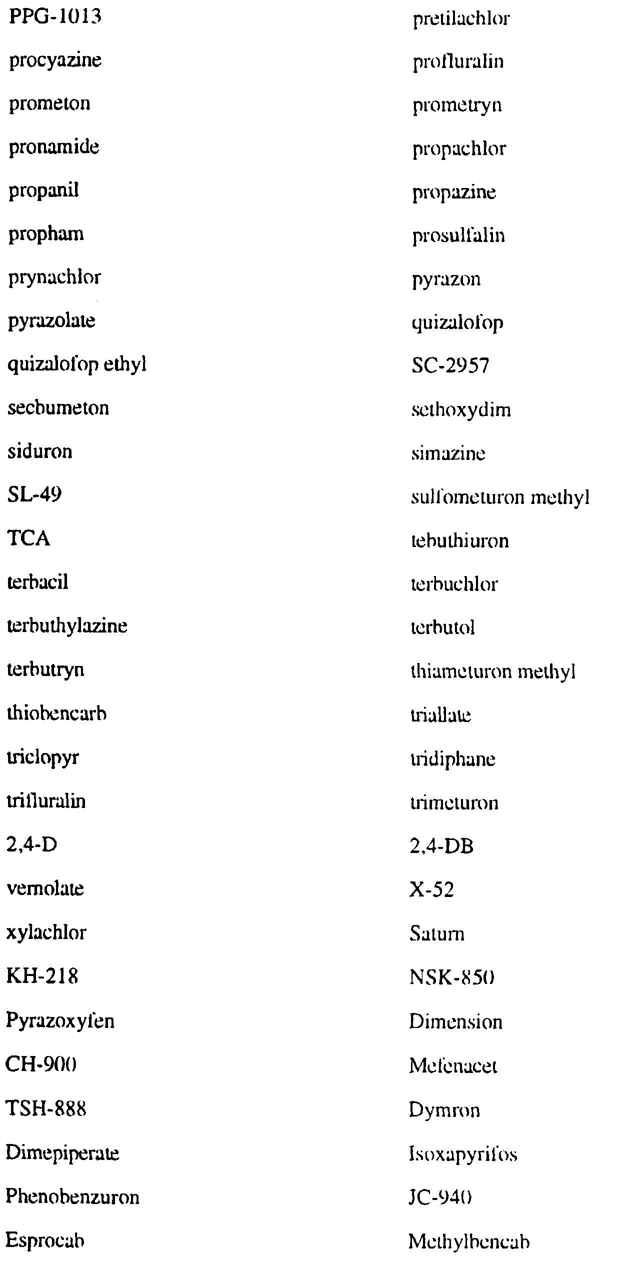

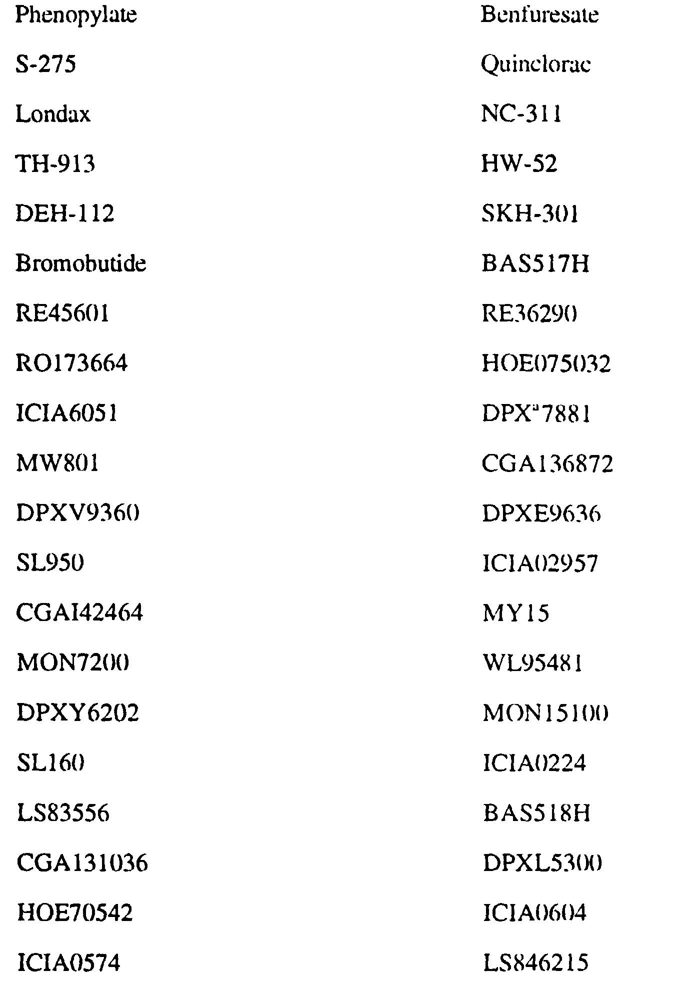

### [Formulation]

Formulations for the use of the compounds of formula( I ) can be prepared in conventional ways. They include dusts, granules, pellets, solutions, suspensions, emulsions, wettable powders, emulsifiable concentrates and the like. Many of these may be applied directly.

Sprayable formulations can be prepared in suitable media and used at spray volumes of from a few liters to several houndred liters per hectare. High strength compositions are primarily used as intermediates for further formulation. The formulations, broadly, contain about 0.1% to 98.9% by weight of active ingredient(s) and at least one of (1) about 0.1% to 20% surfactant(s) and (2) about 1% to 99.8% solid or liquid inert diluent(s) are recommended. More specially, the formulations will contain these ingredients in the following approximate proportions:

**Table 2**

| Formulations | Weight Percent(%) | | |
|---|---|---|---|
| | Active Ingredient | Diluent Surface | Active Agent |
| Wettable Powders | 20-90 | 1-74 | 1-10 |
| Oil Suspension, Emulsions, Solution Emulsifiable Concentrates | 3-50 | 40-95 | 0.1-15 |
| Aqueous Suspension | 10-50 | 40-84 | 1-20 |
| Dusts | 1-25 | 70-98.9 | 0.1-5 |
| Granules and Pellets | 0.1-95 | 5-99.8 | 0.1-15 |
| High strength Composition | 90-98.9 | 1-10 | 0.1-2 |

Lower or higher levels of active ingredient can, of course, be present depending on the intended use and the physical properties of the compound. Higher ratios of surface active agent to active ingredient are sometimes desirable, and are achieved by incorporat ion into the formulation or by tank mixing.

Typical solid diluents are mentioned in the writings of Watkins, et al.("Handbook of Insecticide Dust Diluents and Carrier" 2nd Ed., Dorland Books, Caldwell, N.J.,) and other solid diluents can be used.

The more absorptive diluents are preferred for wettable powders and the denser ones for dusts.

Typical liquid diluents and solvents are mentioned in the writings of Marsden ("Solvents Guide", 2nd Ed., Interscience, New York, 1950).

Solubility under 0.1 % is preferred for concentrated suspension; concentrated solution is preferably stable against phase separation at 0°C.

The surface active agents and their using method is mentioned in the writings of McCutcheon (McCutcheon's Detergents and Emulsifiers Annual, Mc Publishing Corp., Ridgewood, N. J.,) and Sisely et al. (Sisely snd Wood, "Encyclopedia of Surface Active Agents", Chemical Publishing Co., Inc., New York, 1964).

All the above formulations may contain a small amount of additives to reduce foaming, caking, corrosion and the growth of microorganisms.

The preparation methods of such compositions are well known. A solution can be made only by blending properties and a fine solid composition by blending and pulverizing.

Suspension agents can be made by wet milling method (U.S. Patent No. 3,060,084) and granules and pellets can be made by spraying the active ingredient on preformed granular carrier, or by Agglomeration method (J.E. Browing, "Agglomeration "Chemical Engineering, Dec. 4,1967, pp147 / "Perry's Chemical Engineer's Handbook," 5th Ed., Mcgraw-Hill, New York, 1973, pp 8-57ff).

For further information regarding the art of formulations, see for example: US patent No. 3,235,361 / 3,309,192 / 2,891,855, G. C. Klingman, "Weed Control as a Science", John Wiley and Sons, Inc., New York, 1961, pp.81-96 / J. D. Fryer and S. A. Evans, "Weed Control Handbook", 5th Ed., Blackwell Scientific Publications Oxford, 1968, pp.101-103.

The compounds of the present invention can be used independently and may be used in combination with any other commercial herbicides. To specify some more the manufacturing and using of the compounds of the present invention, the detailed examples are described below.

### EXAMPLE 1

### Erythro 2-(1-acetoxy-2-fluoro-n-propyl)-N-t-butyl-benzenesulfonamide.

Erythro *N-t*-butyl-2-(2-fluoro-1-hydroxy-*n*-propyl)-benzenesulfonamide (3.5 g) was dissolved in 50 *ml* of methylene chloride and herein acetic anhydride (1.25 *ml* ), pyridine(1.1 *ml*) and *N,N*-dimethyl aminopyridine(0.12 g) were added. After stirring for 1 hour, the reacting solution was diluted with methylene chloride and washed with 5% hydrochloric acid solution. The seperated organic layer was dried with magnesium sulfate, filtered and concentrated. And then the obtained residue was chromatographed through silicagel using 1 : 3(v/v) solution of ethyl acetate/hexane to afford 3.7 g of the desired product(white solid).
- m.p.: : 134∼135°C
- ¹H NMR(200MHz, CDCl₃): : δ 1.25(s, 9H), 1.36(dd, 3H, *J*_{H-H}=6.4Hz, *J*_{H-F}= 25.3Hz),2.17(s, 3H), 4.86-5.22(m, 1H), 5.47(brs, 1H), 6.68(dd, 1H, *J*_{H-H}=3Hz, *J*_{H-F}=18.6Hz), 7.41-7.71(m, 3H), 8.04-8.12(m, 1H).
- IR(KBr) ν (C=O): 1715 cm⁻¹

Crystal data of product prepared by the above EXAMPLE 1 is the following.

| Crystal data | |
|---|---|
| Molecular Formula | C₁₅ H₂₂FNO₄S |
| Measured Density(Dₘ) | 1.3 Mgm⁻³ |
| Molecular Weight(Mᵣ) | 331.4 |
| Used Wave Length(λ ) | 0.71069 Å |
| Crystal System | monoclinic system |
| No. of diffraction data used in measuring lattic constant | 25 |

Size of unit cell
a = 13.693(6) Å
b = 14.731(15) Å
c = 8.737(5) Å
β = 106.51(5) Å

| | |
|---|---|
| Volume of unit cell (V) | 1690(1) Å |
| Independent Molecularity(Z) | 4 |
| Calculating Density(Dx) | 1.303 Mg m⁻³ |
| Hygroscopic Coefficient(µ ) | 1.74 mm⁻¹ |
| Experimental temperature | 299 K |
| Size of crystal used in measuring | 0.3 x 0.2 x 0.2 mm |
| Color | Colorless |
| Crystal source | obtained on synthesizing |

| Data Collection | |
|---|---|
| Used Diffractometer | CAD-4 diffractometer made in Netherland Enraf-Nonius company |
| Maximum angle of Scan | θ ₘₐₓ = 24° |
| Scanning Method | ω /2θ scans |
| Range of Miller Index | h=-15→15 k=0→16 1=0→9 |
| Absorption Correction Method | did not correct. |
| measuring method | 3 of standard data were confirmed every time diffraction data was measured. |
| Change of standard data on measuring | no change |
| No. of Measured Data | 2549 |
| No. of Independent Data | 2549 |
| No. of measured data in significant having threefold of standard deviation | 2337 [F>30(F)] |

| Refinement | |
|---|---|
| Data used in refining | F |

| Refined parameter | |
|---|---|
| non-hydrogen atom | atomic coordinates x,y,z and anisotropic temperature factor (uᵢⱼ) |
| hydrogen atom | isotropic temperature factor (u) |
| hydrogen atom coupled nitrogen[H(N)] | atomic coordinates x,y,z and isotropic temperature factor (u) |
| No. of parameter refined by the minimum square method | 224 |
| Final Reliancity factor(R) | 0.0598 |
| Sequencity of refining process variables by the minimum square method (S) | 3.5233 |
| Manimum differential-composite electron density(△Pmax) | 0.481 e Å⁻³ |
| Minimum differential-composite electron density(△Pmin) | 0.349 e Å⁻³ |
| No. of data used in refining | 2337 [F>30(F)] |

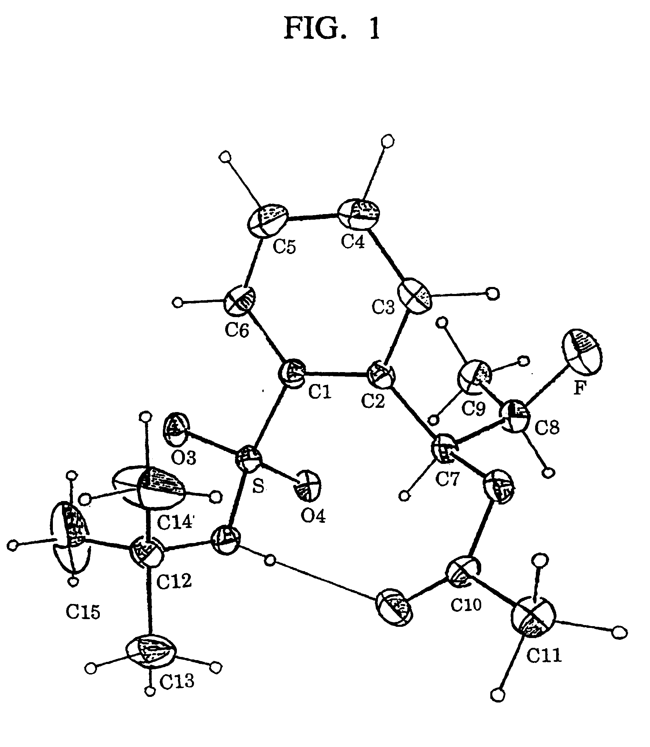

Atomic scattering factor used in X-ray crystalography is described in the Table 3 and stereoconfiguration of innermolecular atoms are given in Figure 1.

**Table 3**

| Atoms | x | y | z | Ueq |
|---|---|---|---|---|
| S | 0.2176(1) | 0.3425(0) | 0.7251(1) | 0.040 |
| F | 0.0756(2) | 0.6606(1) | 0.8577(3) | 0.083 |
| O(1) | 0.2426(2) | 0.6366(1) | 0.7567(3) | 0.052 |
| O(2) | 0.3216(2) | 0.5628(2) | 0.6045(4) | 0.083 |
| O(3) | 0.2209(2) | 0.2488(1) | 0.7683(3) | 0.058 |
| O(4) | 0.1302(1) | 0.3754(1) | 0.6074(2) | 0.051 |
| N | 0.3128(2) | 0.3635(2) | 0.6569(3) | 0.045 |
| C(1) | 0.2312(2) | 0.4071(2) | 0.9026(3) | 0.039 |
| C(2) | 0.2183(2) | 0.5022(2) | 0.9026(3) | 0.037 |
| C(3) | 0.2415(2) | 0.5461(2) | 1.0485(4)) | 0.052 |
| C(4) | 0.2731(3) | 0.4985(3) | 1.1913(4) | 0.061 |
| C(5) | 0.2813(3) | 0.4053(3) | 1.1882(4) | 0.061 |
| C(6) | 0.2616(2) | 0.3593(2) | 1.0443(4) | 0.053 |
| C(7) | 0.1759(2) | 0.5586(0) | 0.7527(4) | 0.043 |
| C(8) | 0.0707(2) | 0.5970(2) | 0.7359(4) | 0.052 |
| C(9) | -0.0067(3) | 0.5269(3) | 0.7416(5) | 0.066 |
| C(10) | 0.3137(3) | 0.6290(2) | 0.6793(4) | 0.050 |
| C(11) | 0.3779(3) | 0.7120(3) | 0.6955(5) | 0.070 |
| C(12) | 0.4207(2) | 0.3314(2) | 0.7215(4) | 0.057 |
| C(13) | 0.4770(3) | 0.3671(4) | 0.6105(5) | 0.097 |
| C(14) | 0.4689(4) | 0.3677(7) | 0.8839(6) | 0.162 |
| C(15) | 0.4217(5) | 0.2279(4) | 0.787(12) | 0.191 |

### EXAMPLE 2

### Threo 2-(1-acetoxy-2-fluoro-n-propyl)-N-t-butyl-benzenesulfonamide

From threo *N-t*-butyl-2-(2-fluoro-1-hydroxy-*n*-propyl)-benzenesulfonamide (6 g) was obtained 6.4 g of the desired product (white solid) using the same method of EXAMPLE 1.
- m.p.: : 126∼127°C
- ¹H NMR(200MHz, CDCl₃): : δ 1.23(s, 9H), 1.36(dd, 3H, *J*_{H-H}=6.4Hz, *J*_{H-F}=23.6Hz), 2.18(s, 3H), 4.73-5.11(m, 1H), 5.54(brs, 1H), 6.49(dd, 1H, *J*_{H-H}=3.8Hz, *J*_{H-F}=21.6Hz). 7.41-7.69(m, 3H), 8.02-8.11(m, 1H).
- IR(KBr) ν (C=O): 1715 cm⁻¹

### EXAMPLE 3

### Erythro 2-(1-acetoxy-2-fluoro-n-propyl)-benzenesulfonamide

Erythro 2-(1-acetoxy-2-fluoro-*n*-propyl)-*N-t*-butyl-benzenesulfonamide (3.7 g) was dissolved in trifluoroacetic acid (20 *ml*) After stirring for 24 hours at room temperature the reaction mixture was concentrated under vacuum and residue solution was diluted with methylene chloride and washed with 5% NaHCO₃ solution.

The organic layer was dried with magnesium sulfate, filtered and concentrated. and then the concentrated solution was column chromatograped using eluate of ethyl acetate/hexane to afford 2.3 g of the desired product (white solid).
- m.p.: : 105∼107°C
- ¹H NMR(200MHz, CDCl₃): : δ 1.33(dd, 3H, *J*_{H-H}=6.4Hz, *J*_{H-F}=24.6Hz), 2.18(s, 3H), 4.85-5.23(m, 1H), 5.55(brs, 2H), 6.53-6.68(m, 1H), 7.46-7.75(m, 3H), 8.06-8.13(m, 1H).

### EXAMPLE 4

### Threo 2-(1-acetoxy-2-fluoro-n-propyl)-benzenesulfonamide

The desired product 3.9 g(white solid) was obtained by the same method of EXAMPLE 3 from threo 2-(1-acetoxy-2-fluoro-*n*-propyl)-*N-t*-butyl-benzenesulfonamide (6.4 g).
- m.p.: : 126∼128°C
- ¹H NMR(200MHz, CDCl₃): : δ 1.36(dd, 3H, *J*_{H-H}=6.4HZ, *J*_{H-F}=24.2Hz), 2.18(s, 3H), 4.75-5.12(m, 1H), 5.57(brs, 2H), 6.38-6.53(m, 1H), 7.46-7.66(m, 3H), 8.06-8.13(m, 1H).

### EXAMPLE 5

### Erythro 2-(1-acetoxy-2-fluoro-n-propyl)-N-[(4,6-dimethoxypyrimidin-2-yl)-aminocarbonyl]-benzenesulfonamide [Compound No. 4]

Erythro 2-(1-acetoxy-2-fluoro-*n*-propyl)-benzenesulfonamide (2.3 g) was dissolved in 20 *ml* of acetonitrile and herein 2.3 g of phenyl (4,6-dimethoxy pyrimidin-2-yl) carbamate was added at room temperature. 1 *ml* of DBU was slowly added dropwised. The reacting solution was stirred for 30 minutes and diluted with 100 *ml* of methylen chloride. Washed with 50 *ml* of 5% hydrochloric acid solution and 50 *ml* of water, the organic layer was dried with magnesium sulfate, filtered and concentrated. The obtained residue was treated with ethyl acetate/hexane/ethylether to afford 2.9 g of the desired product (white solid).
- m.p.: : 191∼193°C
- ¹H NMR(200MHz, CDCl₃): : δ 1.33(dd, 3H, *J*_{H-H}=6.4Hz, *J*_{H-F}=24.6Hz), 2.04(s, 3H), 3.96(s, 6H), 4.86-5.25(m, 1H), 5.80(s, 1H), 6.70-6.82(m, 1H), 7.18-7.70(m, 4H), 8.30-8.40(m, 1H), 13.15(brs, 1H).

### EXAMPLE 6

### Threo 2-(1-acetoxy-2-fluoro-n-propyl)-N-[(4,6-dimethoxypyrimidin-2-yl)amino-carbonyl]-benzenesulfonamide [ Compound No. 5]

5.3 g of the desired product was obtained using the same method of EXAMPLE 5 from 3.9 g of threo 2-(1-acetoxy-2-fluoro-*n*-propyl)-benzenesulfonamide.
- m.p.: : 194∼196°C
- ¹H NMR(200MHz, CDCl₃): : δ 1.33(dd, 3H, *J*_{H-H}=6.4Hz, *J*_{H-F}=24.2Hz), 2.04(s, 3H), 3.96(s, 6H), 4.80-5.14(m, 1H), 5.80(s, 1H), 6.42-6.62(m, 1H), 7.23-7.70(m, 4H), 8.27-8.37(m, 1H), 12.95(brs, 1H).

### EXAMPLE 7

### Erythro N-[(4,6-dimethoxypyrimidin-2-yl)aminocarbonyl]-2-(2-fluoro-1-hydroxy-n-propyl)-benzenesulfonamide [Compound No. 1]

Erythro 2-(1-acetoxy-2-fluoro-*n*-propyl)-*N*-[(4,6-dimethoxypyrimidin-2-yl)amino-carbonyl]-benzenesulfonamide (2.9 g) was dissolved in 60 *ml* of tetrahydrofuran and herein 0.9 g of lithium hydroxide and 10 *ml* of water were added. After stirring for 12 hours at room temperature, the reaction mixture was acidified with hydrochloric acid at 0°C. The reacting solution was diluted with 100 *ml* of ethyl acetate and once washed with water. The organic layer was dried with magnesium sulfate, filtered and concentrated. The obtained residue was treated with ethyl ether and hexane to afford 2.3 g of the desired product. (white solid)
- m.p.: : 166∼168°C
- ¹H NMR(200MHz, CDCl₃): : δ 1.33(dd, 3H, *J*_{H-H}=6.4Hz, *J*_{H-F}=24.6Hz), 3.08(brs, 1H), 3.96(s, 6H), 4.86-5.25(m, 1H), 5.80(s, 1H), 5.89-6.07(m, 1H), 7.36-8.24(m, 5H), 12.82(brs, 1H).
- IR(KBr) ν (C=O): 1705 cm⁻¹

### EXAMPLE 8

### Threo N-[(4,6-dimethoxypyrimidin-2-yl)aminocarbony)]-2-(2-fluoro-1-hydroxy-n-propyl]-benzenesulfonamide [ Compound No. 2]

3.0g of the desired product (white solid) was obtained using the same method of EXAMPLE 7 from threo 2-(1-acetoxy-2-fluoro-*n*-propyl)-*N*-[(4,6-dimethoxypyrimidin-2-yl)aminocarbonyl]-benzenesulfonamide(3.7 g).
- m.p.: : 189∼191°C
- ¹H NMR(200MHz, CDCl₃): : δ 1.36(dd, 3H, *J*_{H-H}=6.4Hz, *J*_{H-F}=24.2Hz), 3.0(brs, 1H), 3.96(s, 6H), 4.78-5.11(m, 1H), 5.80(s, 1H), 5.79-5.91(m, 1H), 7.22-7.78(m, 4H), 8.13-8.22(m, 1H), 12.75(brs, 1H).
- IR(KBr) ν (C=O): 1691 cm⁻¹

### EXAMPLE 9

### Erythro 2-(1-acetoxy-2-fluoro-n-propyl)-3-pyridinesulfonamide.

5.0 g of erythro 2-(1-acetoxy-2-fluoro-*n*-propyl)-*N*-(1,1-dimethylethyl)-3-pyridinesulfonamide was dissolved in 20 *ml* of trifluoroacetic acid. After stirring for 12 hours at 35°C, the reaction solution was concentrated under vacuum. The residue was dissolved in methylene chloride and washed with NaHCO₃ solution. The organic layer was dried with anhydrous magnesium sulfate and the residue was crystallized with ethyl acetate and hexane to afford 3.0 g of the desired product.
- m.p.: : 141∼143°C
- ¹H NMR(200MHz, CDCl₃): : δ 1.55(dd, 3H, *J*_{H-H}=6.5Hz, *J*_{H-F}=25Hz), 2.18(s, 3H), 4.93-5.29(m, 1H), 5.68(brs, 2H), 6.55-6.62(m, 1H), 7.43-7.50(m, 1H), 8.35-8.38(m, 1H), 8.82-8.85(m, 1H)

Crystal data of the product prepared by the above EXAMPLE 9 is the following.

| Crystal Data | |
|---|---|
| Molecular Formula | C₁₀H₁₃FN₂O₄S |
| Crystal System | Trichlinic system |
| Space Group | P1 |
| Molecularity of inner unit lattice(Z) | 2 |
| a = 8.529, b =10.270, c = 8.528, α =110.09, β = 99.28,γ =110.08 | |
| No. of independent diffraction data | 1953 |
| Final Reliancity factor | 6.19% |
| X-ray Wave Length | 1,5405 |

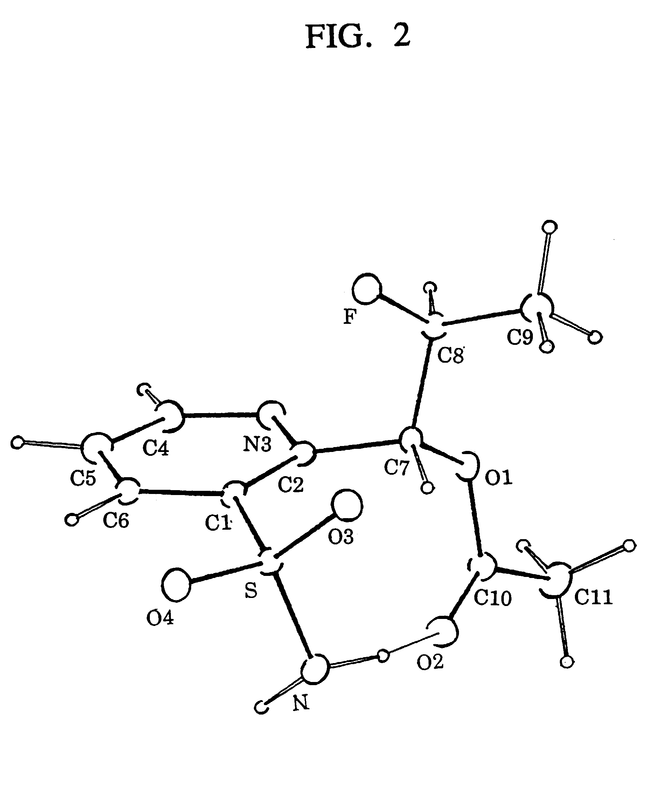

Atomic scattering factor used to X-ray crystalography is described in the following Table 4 and stereoconfiguration of innermolecular atoms are given in Figure 2.

**Table 4**

| Atoms | x | y | z | Ueq |
|---|---|---|---|---|
| S | 0.63350(0) | 0.56940(0) | 0.37580(1) | 0.205(4) |
| F | 0.9981(5) | 0.9201(5) | 0.7942(7) | 0.29(1) |
| N | 0.4564(8) | 0.5644(8) | 0.2715(8) | 0.27(2) |
| O1 | 0.6198(6)) | 0.9893(5) | 0.7386(6) | 0.23(1) |
| O2 | 0.3911(7) | 0.8254(7) | 0.4979(8) | 0.28(1) |
| O3 | 0.7802(7) | 0.6911(6) | 0.3769(7) | 0.28(1) |
| O4 | 0.6179(7) | 0.4174(6) | 0.3044(7) | 0.27(1) |
| C1 | 0.6273(8)) | 0.6136(7) | 0.5963(8) | 0.19(1) |
| C2 | 0.6479(8) | 0.7545(8) | 0.7121(8) | 0.19(1) |
| N3 | 0.6190(8) | 0.7774(7) | 0.8588(7) | 0.22(1) |
| C4 | 0.573(1) | 0.6565(8) | 0.9094(9) | 0.28(2) |
| C5 | 0.561(1) | 0.5174(9) | 0.807(1) | 0.27(2) |
| C6 | 0.5858(9) | 0.4914(8) | 0.6428(9) | 0.23(2) |
| C7 | 0.7158(8) | 0.9012(7) | 0.8863(9) | 0.21(1) |
| C8 | 0.9037(9) | 1.0076(8) | 0.8095(8) | 0.21(2) |
| C9 | 0.994(1) | 1.1395(9) | 0.768(1) | 0.28(2) |
| C10 | 0.4606(9) | 0.9429(8) | 0.6262(9) | 0.24(2) |
| C11 | 0.388(1) | 1.056(1) | 0.689(1) | 0.40(3) |
| HA | 0.4615 | 0.6782 | 0.3215 | 0.0740 |
| HB | 0.3448 | 0.4873 | 0.2893 | 0.3006 |
| H4 | 0.5438 | 0.6717 | 1.0313 | 0.0542 |
| H5 | 0.5324 | 0.4277 | 0.8508 | 0.0636 |
| H6 | 0.5733 | 0.3807 | 0.5557 | 0.0222 |
| H7 | 0.7043 | 0.8652 | 0.5485 | 0.0480 |
| H8 | 0.8978 | 1.0570 | 0.9413 | 0.0785 |
| H9A | 0.9217 | 1.2086 | 0.7800 | 0.4316 |
| H9B | 0.9996 | 1.0959 | 0.6356 | 0.0814 |
| H9C | 1.1261 | 1.2090 | 0.8595 | 0.0846 |
| H11A | 0.2604 | 1.0164 | 0.5974 | 0.1528 |
| H11B | 0.4757 | 1.1654 | 0.6998 | 0.2462 |
| H11C | 0.3748 | 1.0680 | 0.8169 | 0.4053 |

### EXAMPLE 10

### Threo 2-(1-acetoxy-2-fluoro-n-propyl)-3-pyridinesulfonamide

1.6 g of the desired product was obtained using the same method of EXAMPLE 9 from threo 2-(1-acetoxy-2-fluoro-*n*-propyl)-*N*-(1,1-dimethylethyl)-3-pyridinesulfonamide (3.0 g)
- m.p.: : 164∼165°C
- ¹H NMR(200MHz, CDCl₃): : δ 1.17(dd, 3H, *J*_{H-H}=6.5Hz, *J*_{H-F}=23.9Hz), 2.16(s, 3H), 5.03-5.38(m, 1H), 5.79(brs, 2H), 6.54-6.64(m, 1H), 7.43-7.49(m, 1H), 8.35-8.40(m, 1H), 8.80-8.83(m, 1H)

### EXAMPLE 11

### Erythro 2-(1-acetoxy-2-fluoro-n-propyl)-N-[(4,6-dimethoxypyrimidin-2-yl) aminocarbonyl]-3-pyridinesulfonamide [Compound No. 10]

5.1 g of the desired product (white solid) was obtained using the same method of EXAMPLE 5 from 3.9 g of erythro 2-(1-acetoxy-2-fluoro-*n*-propyl-3-pyridinesulfonamide.
- m.p.: : 218∼220°C
- ¹H NMR(200MHz, CDCl₃): : δ 1.46(dd, 3H, *J*_{H-H}=6.4Hz, *J*_{H-F}=24.9Hz), 2.04(s, 3H), 3.96(s, 6H), 4.98-5.26(m, 1H), 5.78(s, 1H), 6.55-6.62(m, 1H), 7.2(brs, 1H), 7.45-7.51(m, 1H), 8.60-8.65(m, 1H), 8.80-8.83(m, 1H), 13.23(br s, 1H)

### EXAMPLE 12

### Threo 2-(1-acetoxy-2-fluoro-n-propyl)-N-[(4,6-dimethoxypyrimidin-2-yl) aminocarbonyl]-3-pyridinesulfonamide [ Compound No. 11]

2.9 g of the desired product (white solid) was obtained using the same method of EXAMPLE 5 from 2.3 g of threo 2-(1-acetoxy-2-fluoro-*n*-propyl)-3-pyridinesulfonamide.
- m.p.: : 190∼192°C
- ¹H NMR(200MHz, CDCl₃): : δ 1.28(dd, 3H, *J*_{H-H}=6.4Hz, *J*_{H-F}=23.9Hz), 2.01(s, 3H), 3.97(s, 6H), 5.08-5.38(m, 1H), 5.79(s, 1H), 6.49-6.60(m, 1H), 7.20(brs, 1H), 7.46-7.53(m, 1H), 8.64-8.69(m, 1H), 8.82-8.85(m, 1H), 13.08(brs, 1H)

### EXAMPLE 13

### Erythro N-[(4,6-dimethoxypyrimidin-2-yl)aminocarbonyl]-2-(2-fluoro-1-hydroxy-n-propyl)-3-pyridinesulfonamide [Compound No. 7]

2.1 g of the desired product (white solid) was obtained using the same method of EXAMPLE 7 from 3.0 g of erythro 2-(1-aceroxy-2-fluoro-*n*-propyl)-*N*-[(4,6-dimethoxypyrimidin-2-yl)aminocarbonyl]-3-pyridinesulfonamide
- m.p.: : 151∼153°C
- ¹H NMR(200MHz, CDCl₃): : δ 1.37(dd, 3H, *J*_{H-H}=6.2Hz, *J*_{H-F}=24.8Hz), 3.95(s, 6H), 4.11(d, 1H), 4.66-4.95(m, 1H), 5.57-5.69(m, 1H), 5.78(s, 1H), 7.33(brs, 1H), 7.46-7.53(m, 1H), 8.62-8.67(m, 1H), 8.79-8.82(m, 1H), 12.98(brs, 1H)

### EXAMPLE 14

### Threo N-[(4,6-dimethoxypyrimidi n-2-yl)aminocarbonyl]-2-(2-fluoro-1-hydroxy-n-propyl)-3-pyridinesulfonamide.[ Compound No. 8]

0.7 g of the desired product (white solid) was obtained using the same method of EXAMPLE 7 from 1.0g of threo 2-(1-acetoxy-2-fluoro-*n*-propyl)-*N*-[(4,6-dimethoxypyrimidin-2-yl)aminocarbonyl]-3-pyridinesulfonamide.
- m.p.: : 173∼175°C
- ¹H NMR(200MHz, CDCl₃): : δ 1.48(dd. 3H, *J*_{H-H}=6.3Hz, *J*_{H-F}=24.2Hz), 3.97(s, 6H), 4.40(d, 1H), 4.90-5.30(m, 1H), 5.31-5.55(m, 1H), 5.82(s, 1H), 7.3(brs, 1H), 7.49-7.55(m, 1H), 8.58-8.63(m, 1H), 8.82-8.85(m, 1H), 13.0(brs, 1H)

### EXAMPLE 15

The herbicidal effect of the compounds of the present invention was tested by the greenhouse test, the method is as followings.

### Pre-emergence test

To produce a suitable preparation of active compound, 1 part by weight of active compound was mixed with 5 parts by weight of acetone, 1 part by weight of alkylaryl polyglycol ether as emulsifier was added and the solution diluted with water to the desired concentration. Seeds of the test plants are shown in normal soil and, after 24 hours, watered with the preparation of the active compound.

It is expedient to keep constant the amount of water per unit area. The concentrat ion of the active compound in the preparation is of no importance, only the amount of active compound applied per unit area being decisive. After three weeks, the degree of damage to the plants was rated in % damage in comparison to the development of the untreated control.

The figures denote :
0% = no action (like untreated control)
20% = slight effect
70% = herbicidal effect
100% = total destruction.

In this test, the active compounds( I ) according to the preparation examples exhibited a better herbicidal activity against mono- and dicotyledon weeds.

### EXAMPLE 16

### post-emergence test

To produce a suitable preparation of active compound, 1 part by weight of active compound was mixed with 5 parts by weight of acetone, 1 part by weight of emulsifier was added and the solution diluted with water to the desired concentration.

Test plants which had a height of 5∼15 cm were sprayed with the preparation of the active compound in such a way as to apply the particular amounts of active compound desired per unit area. The concentration of the spray liquid was so chosen that the particular amounts of active compound desired were applied in 2,0000 *l* of water / ha. After three weeks, the degree of damage to the plants was rated in % damage in comparision to the development of the untreated control.
The figures denote :
0% = no action(like untreated control)
20% = slight effect
70% = herbicidal effect
100% = total destruction.

In this test, the active compounds( I ) according to the preparation examples exhibited a better herbicidal activity against mono- and dicotyledon weeds.

### EXAMPLE 17

### Fresh-water treatment paddy submerged test

A plastic pot having a surface area of 60cm³ or 140cm² was filled with a small amount of fertilizer, after then, the sterilized paddy soil of puddled state at the depth of 5-cm.

Seeds of barnyard grass, umbrella plant, dayflower, monochoria, toothcup, smartweed, and bulrush et al. and perennial nutrition body of flat-sedge and arrowhead et al., were seeded or planted in surface layer of soil, and pregerminated rice with 2-3 leaves was transplanted one root per pot at the depth of 2cm.

After planting, the pot was watered for a day at the depth of 2cm and the manufactured herbicide was spot-treated on the plant in manner sililar to the field condition (4mg/pot).

Two weeks after treatment, herbicidal effect was measured by the same survey standard as that for field condition.

The following Table 5 represents the formula of active ingredients of the present invention. The following Table 6∼8 represents pre- and post-emergence herbicidal effect of active ingredients.

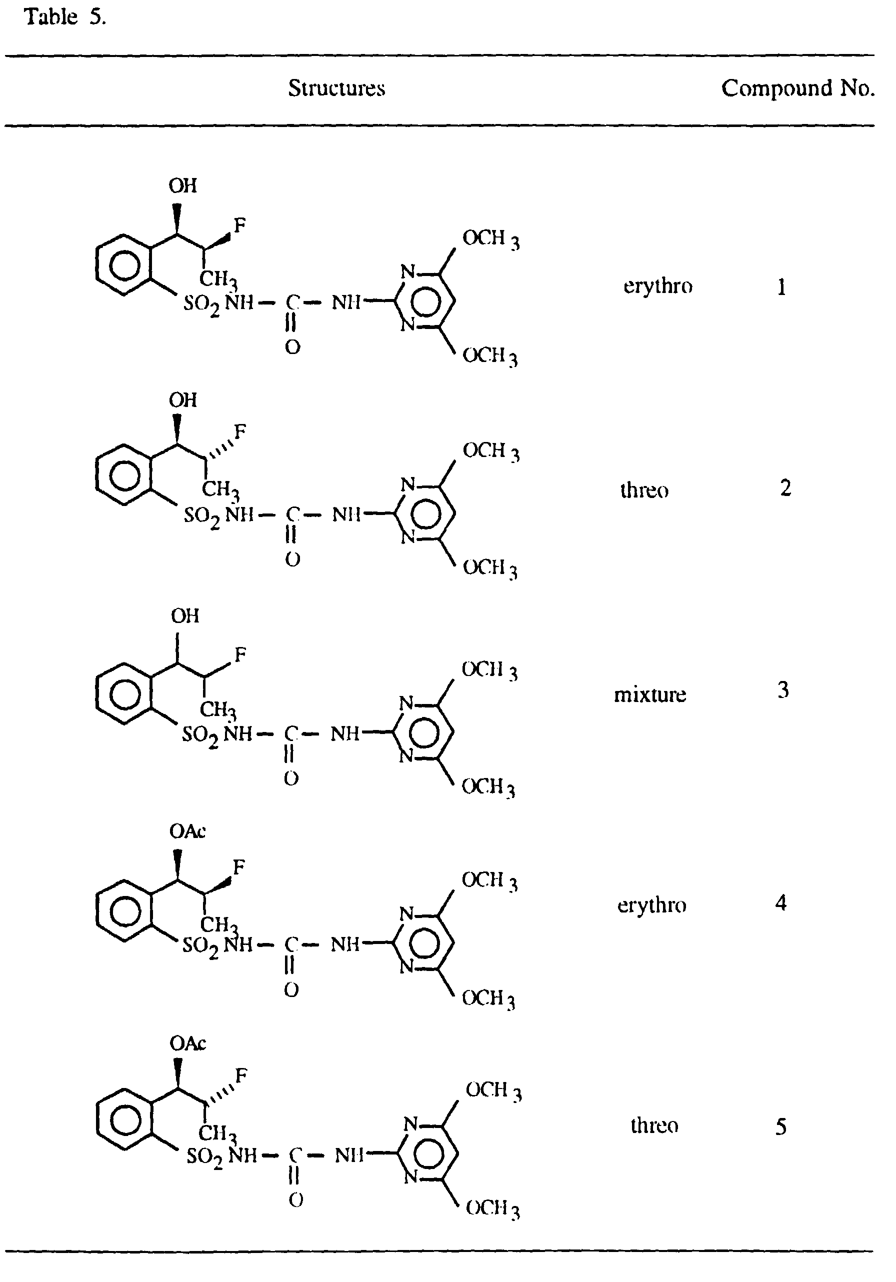

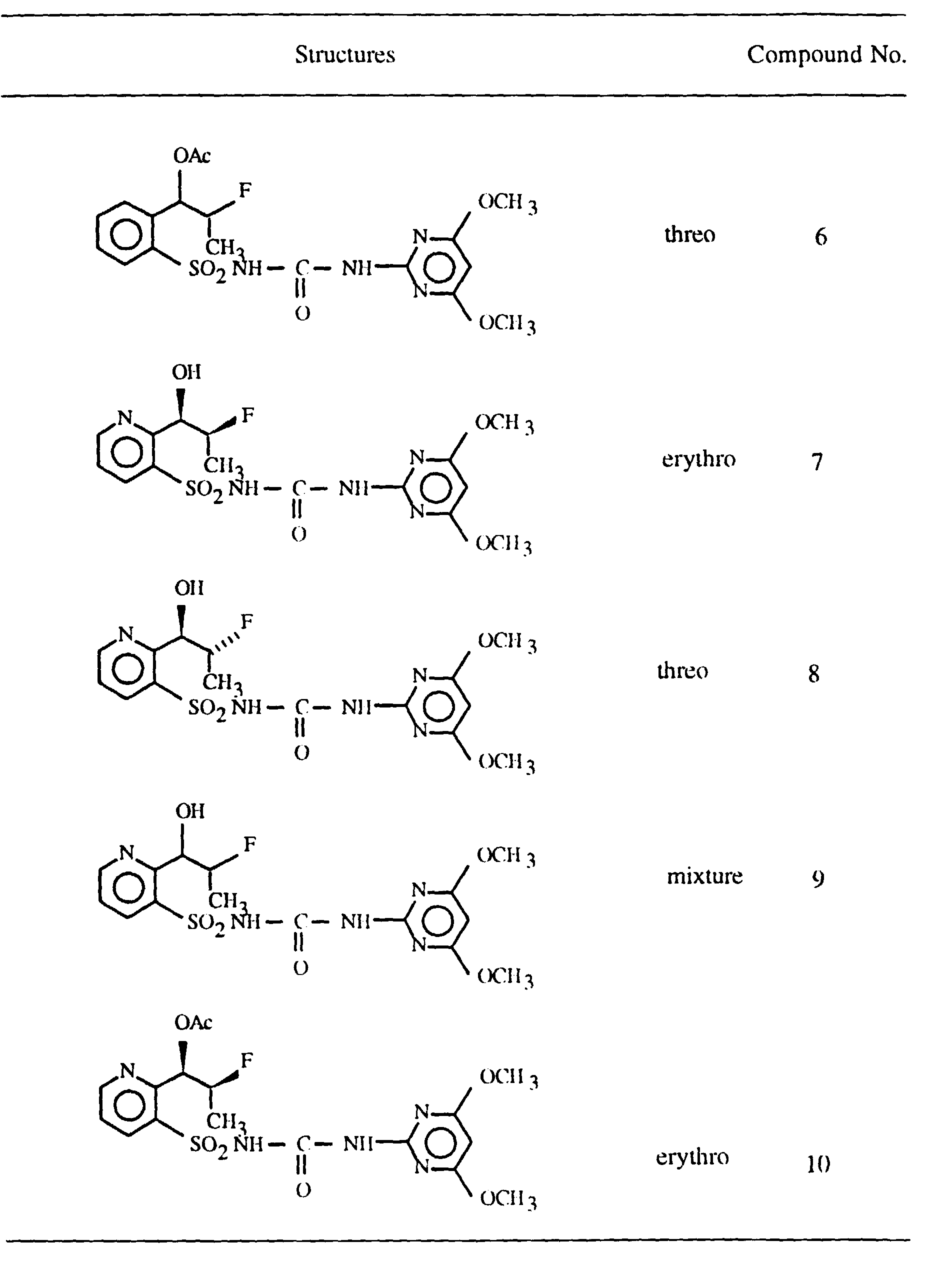

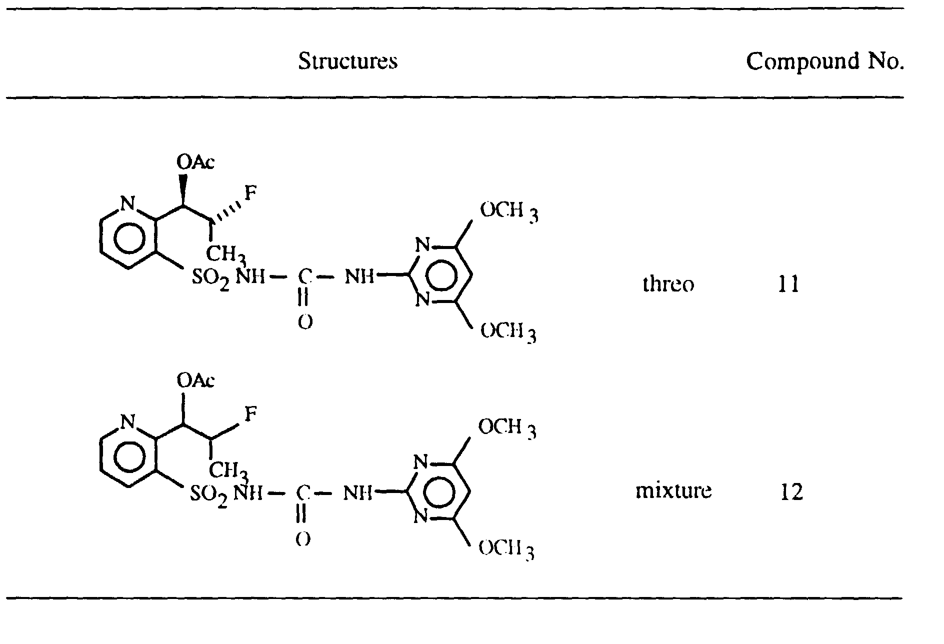

**Table 6**

| PRIMARY SCREENING (PADDY SUBMERGED)-Herbicide | | | | | | | |
|---|---|---|---|---|---|---|---|
| Compound No. | DAT* | kg/ha | ECHOR⁽¹⁾ | SCPJU⁽²⁾ | MOOVA⁽³⁾ | CYPSE⁽⁴⁾ | SAGPY⁽⁵⁾ |
| 1 | 2 | 0.0125 | 100 | 100 | 100 | 100 | 100 |
| 2 | 2 | 0.0125 | 70 | 70 | 100 | 100 | 100 |
| 3 | 2 | 0.0125 | 95 | 80 | 80 | 100 | 60 |
| 4 | 3 | 0.0125 | 95 | 90 | 100 | 85 | 100 |
| 5 | 3 | 0.0125 | 70 | 80 | 70 | 50 | 95 |
| 6 | 2 | 0.0125 | 85 | 80 | 80 | 60 | 75 |
| 7 | 2 | 0.0125 | 100 | 100 | 100 | 100 | 100 |
| 8 | 2 | 0.0125 | 20 | 0 | 40 | 90 | 90 |
| 9 | 2 | 0.0125 | 60 | 40 | 40 | 90 | 100 |
| 10 | 2 | 0.0125 | 100 | 95 | 100 | 100 | 100 |
| 11 | 2 | 0.0125 | 20 | 0 | 50 | 90 | 50 |
| 12 | 2 | 0.0125 | 80 | 30 | 0 | 100 | 100 |

| | | | | | | | |
|---|---|---|---|---|---|---|---|
| (note) *DAT : Day After Treatment | | | | | | | |
| (1)ECHOR : Bchinochloa crus-galli P.BEAUV. var. oryzicolo OHWI. : Barnyard grass | | | | | | | |
| (2)SCPJU : Scirpus juncoides ROXB. : Bulrush | | | | | | | |
| (3) CYPSE : Cyperus serotinus ROTTB. : Flat-sedge | | | | | | | |
| (4) MOOVA : Monochoria vaginalis PRESL. : Monochoria | | | | | | | |
| (5) SAGPY : Sagittaria pygmaca MIQ. : Arrow head | | | | | | | |

**Table 7**

| Harmful Effects Test of Herbicides*¹ | | | |
|---|---|---|---|
| DAT | g/ha | Harmful Effects of Herbicides | |
| | | Compound No.1 | Compound No.7 |
| 5 | 5 | 0 | 0 |
| 5 | 10 | 10 | 0 |
| 5 | 20 | 20 | 0 |

| | | | |
|---|---|---|---|
| * ¹ transplanted rice : 5 DAT treatment after transplanting of 2 leaves rice survey : Comparison of living body weight after herbicidal treatment | | | |

**Table 8**

| Percentage Control for Barnyard grass | | | |
|---|---|---|---|
| Leaf Stage | g/ha | Percentage Control(%) | |
| | | Compound No.1 | Compound No.7 |
| 1 Leaf (6DAS) | 2.5 | 86 | 88 |
| | 5 | 95 | 95 |
| | 10 | 95 | 95 |

## Claims

1. Sulfonyl urea derivatives of the following formula( I ) having substituent of erythro-type stereoisomer, 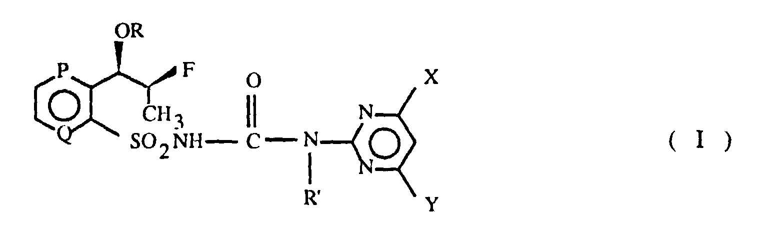 wherein,
P and Q, as equivalent or different group respectively, are CH or N, and present as aromatic ring including P and Q as benzene or pyridine ring ;
R is H, 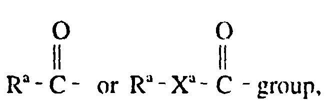 wherein R^{a} is C₁∼C₄ alkyl, C₁∼C₃ haloalkyl, C₂∼C₄ alkenyl or C₂∼C₄ alkynyl group, wherein X^{a} is O, S, NH or NR^{a} group;
R' is H or CH₃ group; and
X and Y are independently halogen atom, C₁∼C₂ alkyl, C₁-C₂ alkoxy or C₁∼C₂ haloalkoxy group.

2. Sulfonyl urea derivatives according to claim 1, wherein said R is hydrogen atom or acetyl group, said P and Q are independently CH or N, and said X and Y are respectively methoxy group.

3. Sulfonyl urea derivative according to claim 1, wherein said formula( I) is erythro *N*-[(4,6-dimethoxy-pyrimidin-2-yl)aminocarbonyl]-2-(2-fluoro-1-hydroxy-*n*-propyl)-benzenesulfonamide.

4. Sulfonyl urea derivatives according to claim 1, wherein said formula( I) is erythro 2-(1-acetoxy-2-fluoro-*n*-propyl)-*N*-[(4,6-dimethoxy-pyrimidin-2-yl)-aminocarbonyl]-benzenesulfonamide.

5. Sulfonyl urea derivatives according to claim 1, wherein said formula( I) is erythro *N*-[(4,6-dimethoxy-pyrimidin-2-yl)aminocarbonyl]-2-(2-fluoro-1-hydroxy-*n*-propyl)-3-pyridinesulfonamide.

6. Sulfonyl urea derivatives according to claim 1, wherein said formula( I) is erythro 2-(1-acetoxy-2-fluoro-*n*-propyl)-*N*-[(4,6-dimethoxy-pyrimidin-2-yl)aminocarbonyl]-3-pyridinesulfonamide.

7. Intermediate compounds of the following formula( II) having erythro-type, 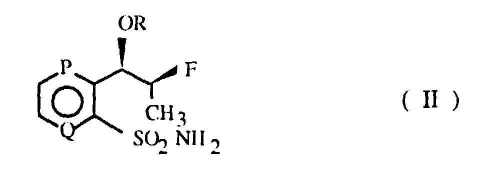 wherein, R,P and Q is respectively as defined in the above claim 1.

8. Intermediate compound according to claim 7, wherein said formula(II) is erythro 2-(1-acetoxy-2-fluoro-*n*-propyl)-benzenesulfonamide.

9. Intermediate compound according to claim 7, wherein said formula(II) is erythro 2-(1-acetoxy-2-fluoro-*n*-propyl)-3-pyridinesulfonamide.

10. Herbicidal compositions including sulfonyl urea derivatives of following formula ( I ) as an effective component. 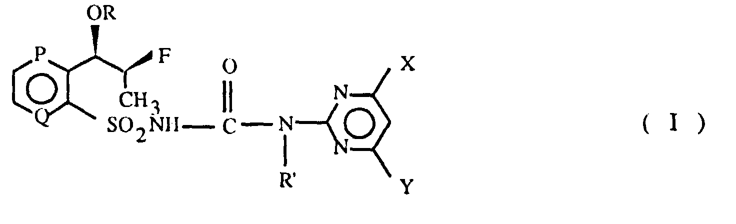 wherein P, Q, R, R', X and Y are respectively as defined in the above claim 1.

11. Herbicidal composition according to claim 10, wherein said sulfonyl urea derivatives of formula( I ) is that R is hydrogen atom or acetyl group; Q is CH; P is CH or N; R' is hydrogen atom; and X and Y are respectively methoxy group.

12. Herbicidal composition according to claim 10, wherein said sulfonyl urea derivatives of formula( I ) is erythro *N*-[(4,6-dimethoxy-pyrimidine-2-yl)-aminocarbonyl]-2-(1-hydroxy-2-fluoro-*n*-propyl)-benzenesulfonamide.

13. Herbicidal composition according to claim 10, wherein said sulfonyl urea derivatives of following formula( I ) is erythro *N*-[(4,6-dimethoxy-pyrimidin-2-yl)aminocarbonyl]-2-(1-hydroxy-2-fluoro-*n*-propyl)-3-pyridine-sulfonamide.

## Patentansprüche

1. Sulfonylharnstoffderivate der folgenden Formel (I) mit einem Substituenten eines Stereoisomers von der erythro-Form 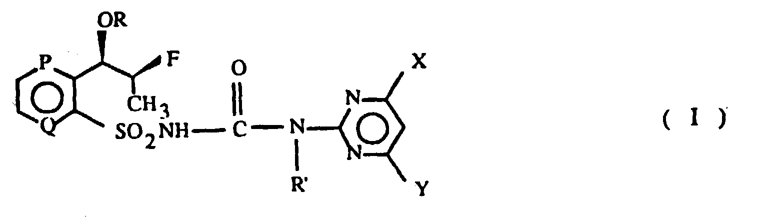 worin
P und Q gleiche oder verschiedene Gruppen CH oder N sind und in einem aromatischen Ring vorliegen, einschließlich P und Q in einem Benzol- oder Pyridinring,
R H, 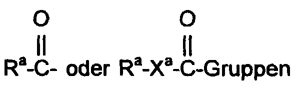 sind, wobei R^{a} eine C₁-C₄-Alkyl-, C₁-C₃-Haloalkyl-, C₂-C₄-Alkenyl- oder C₂-C₄-Alkinylgruppe und X^{a} O, S, eine NH- oder NR^{a}-Gruppe ist,
R' H oder eine CH₃-Gruppe ist und
X und Y unabhängig voneinander Halogenatome, C₁-C₂-Alkyl-, C₁-C₂-Alkoxy- oder C₁-C₂-Haloalkoxygruppen sind.

2. Sulfonylhamstoffderivate nach Anspruch 1, worin R ein Wasserstoffatom oder eine Acetylgruppe ist, P und Q unabhängig voneinander CH oder N und X und Y jeweils Methoxygruppen sind.

3. Sulfonylharnstoffderivate nach Anspruch 1, worin Formel (I) erythro-N-[(4,6-Dimethoxypyrimidin-2-yl)-aminocarbonyl]-2-(2-fluoro-1-hydroxy-n-propyl)-benzensulfonamid ist.

4. Sulfonylharnstoffderivate nach Anspruch 1, worin Formel (I) erythro 2-(1-Acetoxy-2-fluoro-n-propyl)-N-[(4,6-dimethoxypyrimidin-2-yl)-aminocarbonyl]-benzensulfonamid ist.

5. Sulfonylharnstoffderivate nach Anspruch 1, worin Formel (I) erythro N-[(4,6-Dimethoxypyrimidin-2-yl)-aminocarbonyl]-2-(2-fluoro-1-hydroxy-n-propyl)-3-pyridinsulfonamid ist.

6. Sulfonylharnstoffderivate nach Anspruch 1, worin Formel (I) erythro-2-(1-Acetoxy-2-fluoro-n-propyl)-N-[(4,6-dimethoxypyrimidin-2-yl)-aminocarbonyl]-3-pyridinsulfonamid ist.

7. Zwischenprodukte der folgenden Formel (II), welche die erythro-Form aufweisen, 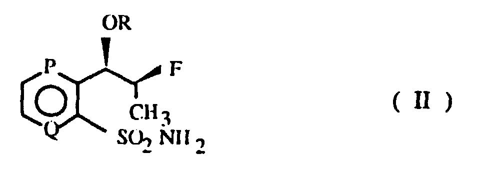 worin R, P und Q wie in Anspruch 1 definiert sind.

8. Zwischenprodukt nach Anspruch 7, worin Formel (II) erythro-2-(1-Acetoxy-2-fluoro-n-propyl)-benzensulfonamid ist.

9. Zwischenprodukt nach Anspruch 7, worin Formel (II) erythro-2-(1-Acetoxy-2-fluoro-n-propyl)-3-pyridinsulfonamid ist.

10. Herbizide Zusammensetzungen, welche Hamstoffderivate der folgenden Formel (I) als einen wirksamen Bestandteil enthalten 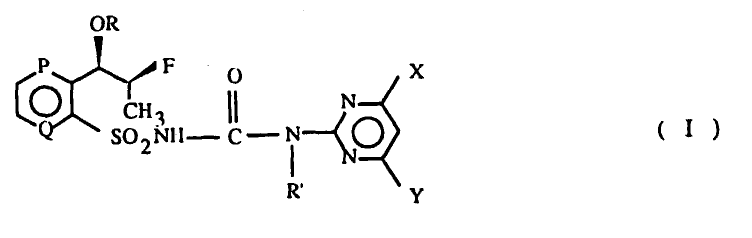 worin P, Q, R, R', X und Y wie in Anspruch 1 definiert sind.

11. Herbizide Zusammensetzung nach Anspruch 10, wobei in den Sulfonylharnstoffderivaten der Formel (I) R ein Wasserstoffatom oder eine Acetylgruppe, Q CH, P CH oder N, R' ein Wasserstoffatom und X und Y jeweils eine Methoxygruppe sind.

12. Herbizide Zusammensetzung nach Anspruch 10, worin das Sulfonylharnstoffderivat nach Formel (I) erythro-N-[(4,6-Dimethoxypyrimidin-2-yl)-aminocarbonyl]-2-(1-hydroxy-2-fluoro-n-propyl)-benzensulfonamid ist.

13. Herbizide Zusammensetzung nach Anspruch 10, worin das Sulfonylharnstoffderivat der Formel (I) erythro-N-[(4,6-Dimethoxypyrimidin-2-yl)-aminocarbonyl]-2-(1-hydroxy-2-fluoro-n-propyl)-3-pyridinsulfonamid ist.

## Revendications

1. Dérivés sulfonylurées ayant la formule suivante (I) comportant un substituant de stéréoisomère du type érythro, 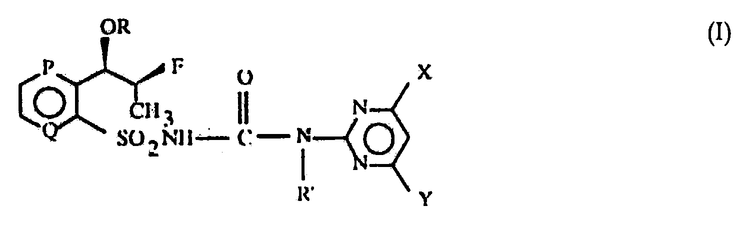 où,
P et Q, étant des groupes identiques ou différents, sont CH ou N, et se présentent sous la forme d'un cycle aromatique incluant P et Q tel que le noyau benzènique ou pyridinique ;
R est H, un groupe 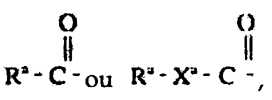 où R³ est un groupe C₁∼C₄ alcoyle, C₂∼C₄ alcoyle halogéné, C₂∼C₄ alcène, ou C₂∼C₄ alcyne, où X³ est O, S, un groupe NH ou NR³ ;
R' est H ou le groupe CH₃ ; et
X et Y sont indépendamment un atome halogène, un groupe C₁∼C₂ alcoyle, C₁∼C₂ alcoolate, C₁∼C₂ alcoolate hydrogéné.

2. Dérivés sulfonylurées selon la revendication 1, dans lesquels ledit R est un atome d'hydrogène ou un groupe acétyle, lesdits P et Q sont indépendamment CH ou N, et lesdits X et Y sont respectivement un groupe méthoxyle.

3. Dérivés sulfonylurées selon la revendication 1, dans lesquels ladite formule (I) est érythro *N*-[(4,6-diméthoxy-pyrimidine-2-yl)aminocarbonyl]2-(2-fluoro-1-hydroxy-*n*-propyl)-benzènesulfonamide.

4. Dérivés sulfonylurées selon la revendication 1, dans lesquels ladite formule (I) est érythro 2-(1-acétoxy-2-fluoro-*n*-propyl)-*N*-[(4,6-diméthoxypyrimidine-2-yl)aminocarbonyl]-benzènesulfonamide.

5. Dérivés sulfonylurées selon la revendication 1, dans lesquels ladite formule (I) est érythro *N*-[(4,6-diméthoxy-pyrimidine-2-yl)aminocarbonyl]-2-(2-fluoro-1-hydroxy-*n*-propyl)-3-pyridinesulfonamide.

6. Dérivés sulfonylurées selon la revendication 1, dans lesquels ladite formule (I) est érythro 2-(1-acétoxy-2-fluoro-*n*-propyl)-*N*-[(4,6-diméthoxypyrimidine-2-yl)aminocarbonyl]-3-pyrimidinesulfonamide.

7. Composés intermédiaires ayant la formule (II) suivante et étant du type érythro 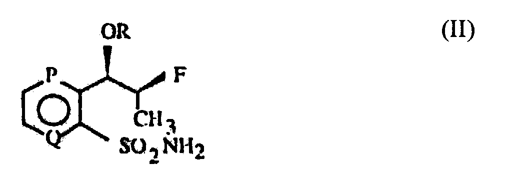 où, R, P et Q sont indépendamment comme défini dans 1a revendication 1 précédente.

8. Composé intermédiaire selon la revendication 7, dans lequel ladite formule (II) est érythro 2-(1-acétoxy-2-fluoro-*n*-propyl)-benzènesulfonamide.

9. Composé intermédiaire selon la revendication 7, dans lequel ladite formule (II) est érythro 2-(1-acétoxy-2-fluoro-*n*-propyl)-3-pyridinesulfonamide.

10. Compositions herbicides incluant des dérivés sulfonylurées de la formule (I) suivante comme composé efficace, 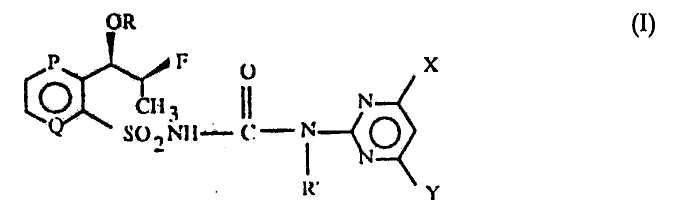 où P, Q, R, R', X et Y sont respectivement comme défini dans la revendication 1.

11. Composition herbicide selon 1a revendication 10, caractérisé en ce que dans lesdits dérivés sulfonylurées de la formule (I), R est un atome d'hydrogène ou un groupe acétyle; Q est CH ; P est CH ou N ; R' est un atome d'hydrogène ; et X et Y sont respectivement un groupe méthoxyle.

12. Composition herbicide selon la revendication 10, dans laquelle lesdits dérivés sulfonylurées de la formule (I) est érythro *N*-[(4,6-diméthoxy-pyrimidine-2-yl)aminocarbonyl]-2-(1-hydroxy-2-fluoro-*n*-propyl)-benzènesulfonamide.

13. Composition herbicide selon 1a revendication 10, dans laquelle lesdits dérivés sulfonylurées de la formule (I) est érythro *N*-[(4,6-diméthoxy-pyrimidine-2-yl)aminocarbonyl]-2-(1-hydroxy-2-fluoro-*n*-propyl)-3-pyridinesulfonamide.
